(19) Europäisches Patentamt
European Patent Office
Office européen des brevets

(11) Numéro de publication: **0 158 564 B1**

(12) # FASCICULE DE BREVET EUROPEEN

(45) Date de publication de fascicule du brevet: **15.07.92**

(21) Numéro de dépôt: **85400598.0**

(22) Date de dépôt: **27.03.85**

(51) Int. Cl.5: **C12N 15/00**, C12P 21/02, A61K 37/64, A61K 35/16, C12N 1/20, C07K 17/10, C12Q 1/56, A61M 1/36, C12N 15/70

(54) **Vecteurs d'expression de l'hirudine, cellules transformées et procédé de préparation de l'hirudine.**

(30) Priorité: **27.03.84 FR 8404755**
**27.08.84 FR 8413250**

(43) Date de publication de la demande:
**16.10.85 Bulletin 85/42**

(45) Mention de la délivrance du brevet:
**15.07.92 Bulletin 92/29**

(84) Etats contractants désignés:
**AT BE CH DE FR GB IT LI LU NL SE**

(56) Documents cités:
**EP-A- 0 094 887**

**CHEMICAL ABSTRACTS; volume 100, no. 19, 7 mai 1984, pge 227, abrégé 152937g, Columbus, Ohio, US; J. DODT et al.: "The complete amino acid sequence of hirudin, a thrombin specific inhibitor. Application of color carboxymethylation", & FEBS Lett. 1984, 165(2), 10-4**

(73) Titulaire: **TRANSGENE S.A.**
**11, rue de Molsheim**
**F-67000 STRASBOURG(FR)**

(72) Inventeur: **Tolstoshev, Paul**
**5, rue Grounod**
**F-67450 Mundelsheim(FR)**
Inventeur: **Harvey, Richard**
**14, avenue Jean Jaurès**
**F-67100 Strasbourg(FR)**
Inventeur: **Courtney, Michael**
**c/o Transgene S.A. 95, rue Saint Lazare**
**F-75009 Paris(FR)**
Inventeur: **Lecocq, Jean-Pierre**
**6, rue du Champ-du-Feu**
**F-67116 Reichsteet(FR)**

(74) Mandataire: **Warcoin, Jacques et al**
**Cabinet Régimbeau 26, avenue Kléber**
**F-75116 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

GENE, volume 13, 1981, Elsevier/North-Holland Biomedical Press, NL; A. HONIGMAN et al.: "Plasmid vectors for positive selection of DNA inserts controlled by the lambda pL promoter repressor and antitermination function"

## Description

La présente invention concerne des vecteurs de clonage et d'expression de la séquence d'ADN codant pour une hirudine ou des analogues de l'hirudine, des microorganismes transformés par ces vecteurs et des procédés permettant d'obtenir par fermentation une hirudine ainsi que l'hirudine obtenue.

L'activité anti-coagulante qui se trouve dans les glandes salivaires des sangsues médicinales, Hirudo medicinalis, provient d'un petit polypeptide que l'on appelle l'hirudine. Cet inhibiteur très spécifique et très efficace de la thrombine a été largement étudié ces derniers temps car il représente potentiellement un agent thérapeutique très intéressant. Toutefois, la difficulté extrème et le coût de son isolation et de sa purification ont empêché qu'il soit utilisé plus largement, ou même qu'il puisse être étudié sur le plan clinique ; le coût de la matière est de l'ordre de 1 300 à 1 600 francs (1983) pour 2 000 U.

La présente invention concerne un moyen pour la production de l'hirudine par le clonage des gènes et leur expression grâce à la technique des ADN recombinants dans une cellule hôte hétérologue afin d'obtenir une grande quantité de polypeptide ayant les propriétés biologiques de l'hirudine.

Le polypeptide avec une activité anti-thrombinique obtenu à partir des glandes salivaires de sangsues a été isolé pour la première fois au milieu de 1950 (1, 2). Cette protéine appelée hirudine a été purifiée à partir des têtes de sangsues environ 650 fois pour obtenir une activité spécifique de 8 500 unités par milligramme.

Le poids moléculaire de la protéine était alors estimé à environ 16 000, cette protéine était stable à la dénaturation par la chaleur et présentait un point isoélectrique de 4,8. Lors d'une électrophorèse sur papier, le produit purifié migre sous forme d'une bande unique et l'analyse d'amino-acide indique que la matière est très riche en amino-acide acide, l'acide aspartique et l'acide glutamique.

Des étapes de purification complémentaires (3) augmentent l'activité spécifique jusqu'à 10 400 U/mg et l'isoleucine a été identifiée comme étant l'amino-acide N-terminal (3, 4).

On a pu démontrer que l'activité de l'hirudine résistait à la digestion avec des enzymes protéolytiques : plasmine, chymotrypsine A et trypsine, mais était sensible à la digestion par la papaïne, la pepsine et la subtilopeptidase A (3).

Le poids moléculaire estimé est maintenant quelque peu inférieur à ce qu'il était dans les documents initiaux, on considère que ce poids moléculaire est de l'ordre de 10 000 daltons et la première estimation de la constante de dissociation du complexe thrombine-hirudine 1:1 ($0{,}8 \times 10^{-10}$) indique une association extrèmement forte entre ces molécules (5). Pour des raisons pratiques, le complexe non-covalent entre ces deux molécules peut être considéré comme non-dissociable in vivo

Le mécanisme d'action de l'hirudine comme anti-coagulant commence seulement à être compris (5). Le substrat pour la fixation de l'hirudine est la thrombine, qui est une enzyme protéolytique, qui, par activation (par le facteur X activé) à partir de sa forme zymogène, la prothrombine, coupe le fibrinogène dans le flux circulatoire pour le transformer en fibrine qui est nécessaire pour la formation du caillot de sang.

L'hirudine est un inhibiteur très spécifique de la thrombine et réagit plus rapidement avec la thrombine que le fibrinogène. En outre, il n'est pas nécessaire d'avoir d'autres facteurs de coagulation ou d'autres constituants du plasma. L'interaction entre les deux molécules est au moins en partie due à leur interaction ionique car l'acétylation des groupes amino libres de la thrombine produit une perte de la fixation d'hirudine. En outre, l'hirudine se fixe dans les mêmes sites que ceux qui sont occupés par la fibrinopeptide et non pas sur les sites actifs de la thrombine car la thrombine acétylée conserve son activité estérase qui n'est pas inhibée par l'hirudine et car la thrombine traitée avec DFP (diisopropyl fluorophosphate) qui phosphoryle les centres actifs de la sérine dans la thrombine continue à fixer l'hirudine.

Le développement suivant dans l'étude de l'hirudine a été la mise au point d'un procédé pour extraire l'hirudine à partir de la sangsue entière (16) au lieu du procédé très délicat consistant à disséquer la tête de l'animal. Le produit final obtenu par ce procédé a une activité biologique similaire à celle de l'hirudine obtenue à partir des têtes mais présente une activité spécifique de 6 500 unités anti-thrombine par milligramme.

La taille estimée de ce composé déterminée par sédimentation a l'équilibre est de 12 000, mais la différence inportante entre cette préparation et les préparations précédentes est que l'on a identifié la valine comme l'amino-acide N-terminal au lieu de l'isoleucine qui avait été trouvée à l'origine. La cause de cette différence avait été apparemment expliquée lorsque l'on a mis en évidence que le second élément de l'extrémité N-terminale se trouvait être de la valine, comme le dansyl dipeptide val-val est résistant à l'hydrolyse acide, on a tout d'abord pensé (7) que l'extrémité N-terminale initiale avec un dipeptide val-val avait été confondue avec le dérivé isoleucine, car ces composants ne sont pas bien résolus par la séparation chromatographique utilisée.

Une préparation d'hirudine à partir de l'animal entier a été utilisée pour déterminer la séquence

d'aminoacide de la protéine (8, 9) qui est représentée à la figure 1. Il apparaît qu'il n'y a pas d'hydrate de carbone attaché à l'hirudine, mais qu'à la position 63 le résidu tyrosine est modifié par un groupe ester O-sulfate.

La fonction de cette modification n'est pas connue mais il est significatif qu'une modification similaire figure également dans le fibrinopeptide B d'un grand nombre d'espèces animales (9).

Une étude récente (10) a démontré que lorsque l'on fait complètement disparaître l'ester sulfate, on réduit l'activité à seulement 55 % de l'activité de l'hirudine initiale.

Le problème de la nature de l'extrémité N de l'hirudine s'est posé à nouveau dans les études (12, 13) qui semblent indiquer que deux formes différentes d'hirudine existent. Une forme à activité faible appelée pseudohirudine, dont on pense qu'elle a été extraite des corps de sangsues, avec la séquence val-val a l'extrémité N-terminale; une forme prédominante dans les têtes qui serait très active et qui présenterait un radical isoleucine à son extrémité N-terminale.

L'activité anti-thrombinique spécifique et très importante de l'hirudine indique immédiatement une application clinique, c'est-à-dire celle d'un anti-coagulant.

L'hirudine a été étudiée de façon très importante chez l'animal pour ses propriétés anti-coagulantes. L'étude la plus détaillée (14) décrit l'activité de l'hirudine dans la prévention des thromboses veineuses, des occlusions vasculaires et des coagulations intra-vasculaires disséminées (DIC) dans le rat. L'hirudine est bien tolérée par les rats, les chiens, les lapins et les souris lorsqu'elle est sous forme très purifiée et injectée par voie intraveineuse. La $DL_{50}$ dans les souris est supérieure,500 000 U/kg de poids corporel (c'est-à-dire 60 mg/kg). Une autre étude (15) indique que les souris tolèrent des doses allant jusqu'à 1 g par kg et que les lapins tolèrent à la fois par voie intraveineuse et par voie subcutanée jusqu'à 10 mg/kg. Dans les souris, des injections répétées pendant une période de deux semaines ne conduisent pas à des réactions de sensibilisation. Deux autres études indépendantes, l'une utilisant les chiens (16) et l'autre (17) démontrant l'activité de l'hirudine dans la prévention des DIC dans les rats, tombent en accord avec les résultats positifs de Markwardt et de ses collaborateurs.

On a aussi pu démontrer que l'hirudine prévient les endotoxines induits par les DIC dans les cochons et ainsi constitue une solution potentielle aux problèmes très sérieux posés par les endotoxinémies qui conduisent à une mortalité élevée chez les cochons. En outre, l'hirudine chez l'animal expérimental est rapidement éliminé (demi-vie de l'ordre de 1 heure) encore sous forme biologiquement active par l'intermédiaire des reins.

Cette étude suggère que l'hirudine peut constituer un agent clinique intéressant comme anti-coagulant. En outre, la pré-phase de coagulation du sang n'est pas affectée compte tenu de la haute spécificité de l'action de l'hirudine, l'activité anti-thrombinique est dépendante de la dose et l'effet de l'hirudine est rapidement reversible compte tenu de son élimination rénale rapide. On a pu mettre en évidence que l'hirudine est très supérieur à l'héparine pour le traitement des DIC (14, 17) comme cela pouvait être attendu compte tenu du fait que la DIC est accompagnée par une décroissance de l'anti-thrombine III (un co-facteur nécessaire pour l'action de l'héparine) et un relarguage du facteur de plaquette 4 qui est un agent très efficace anti-héparine.

L'une des études a mis en évidence la possibilité que l'hirudine puisse être absorbée par la peau des êtres humains (19) bien que les résultats obtenus restent quelque peu difficiles à interpréter.

Des préparations commerciales d'extraits de sangsues bruts sont disponibles (Hirucrème, Exhirud-Blutgel), mais des tests complémentaires avec des doses plus importantes d'une matière hautement purifiée sont nécessaires pour établir s'il s'agit là d'une voie d'administration intéressante.

De façon générale, les voies d'administration préférées sont les voies intra-veineuses, intra-musculaires et la voie percutanée. D'autres voies d'administration ont été rapportées pour l'hirudine, notamment la voie orale (BSM 3 792 M).

Ce produit peut également, en combinaison avec d'autres composants, être utilisé dans le traitement du Psoriasis et d'autres désordres cutanés du même type comme cela est décrit dans le DOS 2 101 393.

L'hirudine peut être en outre utilisée à titre d'anti-coagulant dans les tests cliniques en laboratoires et comme outils de recherche. Là, la haute spécificité pour une étape unique dans la coagulation du sang peut présenter un avantage considérable par rapport aux anti-coagulants utilisés le plus généralement avec une action qui est beaucoup moins spécifique.

En outre, l'hirudine peut être très utile comme agent anti-coagulant dans les circuits extra-corporels et dans les systèmes de dialyses où elle peut présenter des avantages considérables par rapport aux autres anti-coagulants, en particulier s'il peut être immobilisé sous forme active sur la surface de ces systèmes circulatoires artificiels.

Finalement, l'utilisation de l'hirudine marquée peut constituer une méthode simple et efficace pour mesurer les taux de thrombine et de prothrombine.

En résumé, l'hirudine présente un grand nombre d'applications possibles :

1°) comme anti-coagulant dans des conditions thrombotiques critiques pour la prophylaxie et la prévention de l'extension des thromboses existentes ;

2°) comme anti-coagulant pour réduire les hématomes et les enflures après les micro-chirurgies, car il est fait une utilisation importante de sangsues vivantes ;

3°) comme anti-coagulant dans des systèmes de circulation extra-corporelles et comme agent anti-coagulant pour revêtir des biomatériaux synthétiques ;

4°) comme anti-coagulant dans les tests cliniques des échantillons de sang dans les essais de laboratoires ;

5°) comme anti-coagulant dans la recherche clinique sur la coagulation et comme outil expérimental ;

6°) comme agent topique possible pour l'application cutanée dans le traitement des hémorroïdes, des varices et des oedèmes ;

7°) comme composant dans le traitement des psoriasis et autres désordres apparentés.

Enfin, l'hirudine peut être utilisée pour fixer la thrombine dans des milieux où celle-ci est génante (dosage, expérience ou sang traité par exemple). En particulier, l'hirudine permet de limiter les coagulations dans les circuits extra-corporels.

L'hirudine marquée peut, en outre, être utilisée pour détecter la formation de caillots. En effet, la formation de caillots impose la transformation de la prothrombine circulante en thrombine sur laquelle vient se fixer sélectivement l'hirudine ; la détection d'une accumulation d'hirudine marquée en un point du corps du patient permet de visualiser la formation d'un caillot.

En dépit de ces nombreux avantages comme anti-coagulant, l'hirudine n'a, jusqu'à maintenant, pas été utilisée largement même dans la recherche clinique. Ceci tient au fait que la matière naturelle est très difficile à obtenir sous forme pure et par dessus tout qu'elle est particulièrement chère, même pour commencer des essais cliniques afin de mettre en évidence une utilité potentielle. Bien que les procédés de purification adéquats existent (20, 21) pour obtenir des échantillons très purs, la difficulté d'obtention de la matière de base (des sangsues) en quantité suffisante demeure l'obstacle majeur.

Bien que l'hirudine soit commercialement vendue par différentes compagnies (Sigma, Plantorgan, Pentopharm), de telles préparations présentent une activité qui peut varier énormément et des puretés très variables.

C'est pourquoi la production d'hirudine par technologie de l'ADN recombinant est une solution particulièrement attrayante pour obtenir cette matière en grande quantité a des coûts raisonnables afin de permettre de tester et d'utiliser ce type de produit.

Dans ce qui va suivre, on utilisera la plupart du temps la terminologie "l'hirudine". Compte tenu de ce qui vient d'être dit et d'autres éléments qui sont apparus lors de la présente étude, il est clair qu'il existe plusieurs formes d'hirudine et donc que la terminologie "une hirudine" serait plus correcte.

C'est pourquoi, dans ce qui suit on entendra par "hirudine" l'une quelconque des formes de l'hirudine naturelle ou synthétique, c'est-à-dire un produit ayant la même activité in vivo que l'hirudine qui sera parfois nommé "analogue de l'hirudine".

Il convient d'ailleurs de remarquer que les produits dénommés "analogues de l'hirudine" obtenus à partir des bactéries seront dépourvus de la fonction ester O-sulfate, mais par contre que "l'hirudine bactérienne pourra comporter à l'extrémité N-terminale un amino-acide méthionine qui ne figure pas dans l'hirudine native. Mais il est bien entendu que par "analogue de l'hirudine" on entend également protégé des produits d'origine biologique ayant été modifiés après leur production notamment par réaction chimique ou réaction enzymatique.

La présente invention concerne de nouveaux vecteurs de clonage et d'expression dans une cellule hôte de l'hirudine ou d'un analogue de l'hirudine, caractérisés en ce qu'ils comportent le gène codant pour l'hirudine ou un analogue de l'hirudine et les éléments d'expression de ce gène dans ladite cellule hôte.

La nature des éléments d'expression peut varier selon la nature de la cellule hôte. Ainsi, dans les cellules bactériennes, les éléments d'expression comporteront au moins un promoteur bactérien et un site de fixation des ribosomes (qui constitue ce qui sera parfois nommé région codant pour l'initiation de la traduction).

De façon générale, les vecteurs selon la présente invention comporteront, outre le gène codant pour l'hirudine :

- l'origine de réplication d'un plasmide bactérien,
- un promoteur, en particulier tout ou partie d'un promoteur du bactériophage λ : $P_L$, $P_R$ ou $P'_R$ ;
- une région codant pour l'initiation de la traduction incorporant l'ATG soit de l'extrémité 5' du gène de l'hirudine, soit de l'extrémité 5' du gène de l'hirudine fusionné en 5' avec une autre protéine ; la raison de cette fusion est de permettre l'expression d'une protéine de haut poids moléculaire qui se dégrade

moins dans E. coli.

La présence d'une origine de réplication pour un plasmide est essentielle pour permettre la réplication du vecteur dans les cellules bactériennes correspondantes, en particulier dans le cas de E. coli on utilisera, de préférence, l'origine de réplication du plasmide pBR322. Le plasmide pBR322 présente, en effet, l'avantage de donner un grand nombre de copies et ainsi d'augmenter la quantité de plasmides produisant la protéine désirée.

Parmi les promoteurs du bactériophage λ, on utilisera, de préférence, le promoteur principal de gauche noté $\lambda P_L$.$P_L$ est un promoteur puissant responsable de la transcription précoce de λ.

Il est également possible d'utiliser d'autres promoteurs du bactériophage λ, notamment le promoteur de droite, $P_R$ ou le second promoteur de droite, $P'_R$.

Bien qu'il soit possible d'utiliser des séquences d'initiation de la traduction très variées, on préfère utiliser tout ou partie du site de fixation des ribosomes de la protéine cII du bactériophage λ qui sera nommée ci-après λ cIIrbs.

Comme cela sera démontré il est également possible d'utiliser de telles séquences synthétiques en particulier tout ou partie de la séquence :

ATAACACAGGAACAGATCT$\overline{\text{ATG}}$.

Le vecteur en cause comporte, en outre, de préférence une fonction d'antiterminaison de transcription codée par ex. par le gène N de λ noté λN. En présence du produit de transcription du gène N la transcription à partir de $P_L$ se poursuit au-delà de la plupart des signaux stop.

Ceci écarte les problèmes posés par un arrêt prématuré de la transcription qui peuvent se produire lorsque les gènes étrangers clonés présentent de tels signaux stop. En outre, il a été démontré que l'expression à partir de $P_L$ est améliorée dans un environnement $N^+$.

Afin d'écarter les problèmes de toxicité et d'instabilité du système hôte-vecteur en cas de production en continu de grandes quantités d'une protéine étrangère, il est nécessaire de prévoir le contrôle de l'activité du promoteur en lui adjoignant tout ou partie d'un système d'expression inductible, en particulier thermoinductible.

De préférence, le contrôle par la température de la synthèse de la protéine étrangère est effectué au niveau de la transcription au moyen d'un répresseur thermosensible codé dans la bactérie hôte, par exemple cI857, qui réprime l'activité de $P_L$ à 28°C mais qui est inactivé à 42°C. Le répresseur agit sur l'opérateur $O_L$ qui est adjacent au promoteur $P_L$. Bien que dans le cas précédent une partie du système d'expression thermoinductible soit partie intégrante de la bactérie hôte, il est possible de prévoir que ce système fasse partie du vecteur lui-même.

Le vecteur en cause peut également comporter un gène de résistance à un antibiotique, par exemple l'ampicilline dans le cas de pBR322, mais d'autres gènes de résistance peuvent être utilisés, résistance à la tétracycline ($Tet^r$) ou au chloramphénicol ($Cm^r$).

L'incorporation d'un tel marqueur est nécessaire pour la sélection des bactéries contenant les transformants porteurs du plasmide selon l'invention pendant les expériences de clonage.

L'incorporation d'un gène de résistance permet d'augmenter la stabilité du plasmide en imposant une pression de sélection lors de la fermentation, et en outre facilite l'isolement des transformants.

Pour le clonage, il est intéressant de disposer d'un système permettant de détecter l'insertion dans un plasmide d'un ADN étranger.

A titre d'exemple, il est possible de prévoir dans la zone de clonage le fragment N-terminal de la β-galactosidase de E. coli (lacZ') en le fusionnant avec la région d'initiation de traduction dérivée de λcII, ce qui met la traduction du fragment α sous le contrôle des séquences de cII.

Le fragment α est complémenté par l'expression du fragment ω C-terminal codé dans l'hôte, ceci conduit à une activité β-galactosidase dans les cellules. Cette activité β -galactosidase produit des colonies bleues en présence d'un substrat chromophorique, le 5-bromo-4-chloro-3-indolyl-D-galactosidase.

A 28°C, le promoteur $P_L$ est inactivé, le fragment α n'est pas synthétisé et les colonies demeurent blanches. Lorsque la température est amenée à 42°C, le promoteur $P_L$ est activé, le fragment α est synthétisé et les colonies virent au bleu.

L'insertion d'ADN étranger dans les sites de clonage situés dans ce système de détection empêche la synthèse de la β-galactosidase et conduit donc à des colonies blanches aussi bien à 28°C qu'à 42°C. Il est également possible de remplacer le gène lacZ' par d'autres gènes permettant une détection.

Parmi les différentes hirudines qui peuvent être préparées selon l'invention et utilisées, il faut citer :

a) l'hirudine correspondant à une modification du variant 2 dans lequel la séquence N-terminale val-val a été remplacée par ile-thr ;

b) l'hirudine correspondant au variant 1 (dit HV2) dont la structure correspond à celle représentée à la figure 18 b.

La structure des gènes correspondants peut être déduite de celle des amino-acides, comme cela sera démontré dans les exemples ; ces gènes peuvent être synthétisés par l'une quelconque des méthodes connues pour la préparation des ADN synthétiques.

De préférence, on utilisera l'hirudine dont la structure N-terminale correspond à ile-thr ; différentes structures du gène correspondant apparaîtront dans les exemples.

La présente invention concerne les cellules,en particulier les bactéries, notamment les souches de E.coli transformées par les vecteurs selon l'invention par des techniques connues et dont certaines seront rappelées dans les exemples.

Enfin, l'invention concerne un procédé de préparation de l'hirudine ou de ses analogues dans lequel on cultive sur un milieu de culture des bactéries transformées comme décrit précédemment et dans lequel on récupère ensuite l'hirudine ou l'analogue formés.

Les milieux de culture mis en oeuvre sont connus de l'homme de métier et devront être adaptés à chaque souche cultivée. La culture sera, de préférence, effectuée en présence de l'antibiotique à l'encontre duquel la souche transformée est devenue résistante.

L'hirudine ou ses analogues peuvent être purifiés ou pré-purifiés à partir du mélange de fermentation par chauffage à pH acide, notamment à 50-80° C à pH compris entre 1 et 3, en particulier a 70° C à pH 2,8, et récupération du surnageant dans lequel se trouve l'hirudine.

Il est également possible de mettre à profit l'affinité de l'hirudine pour la thrombine en utilisant une résine sur laquelle est fixée la thrombine, par passage du mélange contenant l'hirudine sur une telle résine on fixe l'hirudine qui peut être ensuite éluée par une solution contenant un agent compétiteur de l'hirudine.

La présente invention concerne enfin l'hirudine ou ses analogues obtenus par la mise en oeuvre du procédé selon l'invention, c'est-à-dire l'hirudine ou ses analogues d'origine bactérienne mais également les hirudines ou analogues obtenus à partir de produits bactériens par réaction chimique ou enzymatique, par exemple par clivage chimique ou enzymatique ou bien par réaction chimique ou enzymatique destinée à fixer le radical $-SO_3H$, à l'exclusion de l'hirudine obtenue par culture d'une cellule transformée par un vecteur comportant un gène de l'hirudine identique à celui contenu dans le plasmide pTG726.

En particulier, l'invention concerne les peptides comportant tout ou partie de la formule suivante :

```
                                    ATT ACT TAC ACT GAT TGT ACA GAA TCG GGT CAA AAT TTG TGC CTC TGC GAG GGA AGC AAT GTT TGC GGT
                                    Ile Thr Tyr Thr Asp Cys Thr Glu Ser Gly Gln Asn Leu Cys Leu Cys Glu Gly Ser Asn Val Cys Gly

AAA GGC AAT AAG TGC ATA TTG GGT TCT AAT GGA AAG GGC AAC CAA TGT GTC ACT GGC GAA GGT ACA CCG AAC CCT GAA AGC CAT AAT AAC
Lys Gly Asn Lys Cys Ile Leu Gly Ser Asn Gly Lys Gly Asn Gln Cys Val Thr Gly Glu Gly Thr Pro Asn Pro Glu Ser His Asn Asn

GGC GAT TTC GAA GAA ATT CCA GAA GAA TAT TTA CAA
Gly Asp Phe Glu Glu Ile Pro Glu Glu Tyr Leu Gln
```

Ce peptide (HV2) est représenté avec la séquence codante correspondante qui ne fait pas partie du peptide.

L'invention concerne également les variants de l'hirudine HV2 tels que représentés à la figure 18.

L'invention concerne, enfin, des compositions pharmaceutiques contenant l'hirudine ou ses analogues à titre de principe actif.

Ces compositions peuvent être applicables par voie intra-péritonéale, intra-veineuse, intra-musculaire ou sous-cutanée, par voie orale ou par voie cutanée topique.

Ces compositions contiennent les excipients connus en la matière et éventuellement d'autres principes actifs.

La présente invention comporte, bien entendu, d'autres aspects, notamment certains plasmides qui seront décrits dans les exemples ainsi que leurs mutants et dérivés et de façon générale les procédés de fermentation des bactéries transformées ainsi que les produits ainsi obtenus.

D'autres caractéristiques et avantages de l'invention seront mieux compris à la lecture des exemples ci-après et des dessins annexés sur lesquels :

- la figure 1 représente la séquence d'amio-acide de l'hirudine extraite de la sangsue totale,
- la figure 2 représente la sonde oligonucléotide 48 mer conçue pour le criblage,
- la figure 3 représente la séquence de l'hirudine du clone pTG717,
- la figure 4 représente une autoradiographie du mélange d'hybridation entre la sonde marquée et diverses restrictions de pTG717,
- la figure 5 représente la carte de restriction de l'insert dans pTG717,
- la figure 6 représente le détail de la construction des deux vecteurs d'expression de l'hirudine pTG718 dérivé de pTG927 et pTG719 dérivé de pTG951,
- la figure 7 représente le graphique de l'évolution de l'activité en hirudine des extraits de E. coli en

fonction du temps pour les deux vecteurs,

- la figure 8 représente l'analyse des extraits de E. coli marqués contenant l'activité hirudine,
- la figure 9 représente l'analyse des mêmes extraits qu'à la figure 8 mais après traitement par la chaleur à pH acide.

Il convient de remarquer que les différentes séquences de nucléotides figurant dans les dessins doivent être considérées comme faisant explicitement partie de la présente description, ces séquences n'ont pas été reproduites dans le corps du texte afin de ne pas l'alourdir inutilement.

La plupart des techniques mises en oeuvre dans les exemples suivants sont connues de l'homme de métier et certaines sont décrites dans les références ci-annexées. Seules les techniques particulières seront donc décrites dans le cours de la description. On donnera uniquement à titre de renseignements généraux les caractéristiques des souches mises en oeuvre

a) Souches bactériennes

Les souches bactériennes utilisées dans le cadre de la présente invention sont les suivantes :

- TGE900 qui est une souche de E. coli ayant les caractéristiques suivantes : $su^{-}F^{-}$his ilv bio (λcI857ΔBamΔHI)
- N6437, souche de E. coli ayant les caractéristiques suivantes : $F^{-}$his ilv $gal^{+}$ $\Delta 8proC^{+}$ : tn10 lacΔm15 (λcI857ΔBamΔHI).
- Jm103 qui est une souche de E. coli ayant les caractéristiques suivantes : Δ(lac-pro) $sup^{E}$ thi endA sbcB15 sttA $rK^{-}$ $nK^{+}$ / $F^{1}$ traD36 $\overline{proAB}^{+}$ $laci^{a}$ lacZΔm15.

Les souches mentionnées précédemment ont été utilisées parce qu'elles étaient disponibles, mais il est bien entendu qu'il est possible d'utiliser d'autres souches dans la mesure où elles présentent certaines caractéristiques essentielles qui sont rappelées dans le cours de la description détaillée.

Les exemples ci-après comportent essentiellement étapes suivantes :

1°) la préparation des mARN et la constitution d'une banque de cADN ;

2°) la réalisation d'une sonde ;

3°) la sélection de la banque grâce à cette sonde et l'identification d'un plasmide portant la séquence codant pour l'hirudine ;

4°) la réalisation d'un vecteur d'expression du gène codant pour l'hirudine ;

5°) L'étude des résultats obtenus.

Préparation de l'ARN

Des sangsues vivantes de l'espèce Hirudo médicinalis sont prélevées dans la région de Bordeaux en France. On les fait jeuner pendant un minimum de 4 semaines puis elles sont décapitées et les têtes sont immédiatement congelées dans l'azote liquide. L'ARN de têtes totales est extrait par broyage des têtes sous forme d'une poudre fine dans l'azote liquide. A 1 g de cette poudre on ajoute 5 ml d'un tampon NETS (10 mM Tris HCl, pH 7,5, 100 mM NaCl, 1mM EDTA, O,5 % SDS) et 5 ml de phénol redistillé tous les deux préchauffés à 95°C. La solution est alors mélangée par vortex, centrifugée à 30 000 g, la phase aqueuse est réextraite avec 1 ml de tampon NETS, et les phases aqueuses rassemblées sont réextraites avec 5 ml de phénol frais. L'ARN est précipité par addition de deux volumes déthanol et rassemblé par centrifugation après avoir été abandonné à -20°C pendant plus de 4 heures. Le culot brun foncé est dissout dans 2,5 ml d'eau distillée, 2,5 ml de LiCl 5 M sont ajoutés, et après mélange la solution est abandonnée pendant une nuit à 4°C. La solution est ensuite réhaussée avec 5 ml de LiCl 3 M, et centrifugée à 20 000 g pendant 10 minutes à 4°C. Le surnageant est éliminé et le culot d'ARN clair est repris dans 1 ml de $H_2O$. On le reprend dans NaCl 0,25 M et l'ARN est précipité avec 2 volumes d'éthanol. Le culot d'ARN final est récupéré par centrifugation puis est dissout et stocké à -80°C dans l'eau distillée. La concentration de l'ARN est mesurée par son absorption à 260 nm.

L'ARN messager polyadénylé est préparé en utilisant une cellulose oligo dT et une procédure standard (22). Le rendement en mARN représente environ 2 à 5 % de l'ARN total de départ.

Préparation de l'ADN complémentaire et Construction de la banque de cDNA dans le plasmide vecteur pBR 322.

Le processus mis en oeuvre est exactement le même que celui qui a conduit à la construction de la banque de mARN de foie humain décrite dans la référence 23. Les mARN de têtes de sangsues sont copiées sous forme d'ADN en utilisant un "primer" oligo dT et l'enzyme reverse transcriptase. Un second

brin d'ADN est synthétisé en utilisant l'ADN polymérase, l'épingle à cheveux est ouverte avec la nucléase SI et le cADN double brin est purifié sur un gradient de sucrose. Des cADN de taille déterminée sont terminés à l'extrémité 3' avec des petites extensions de dC en utilisant l'enzyme terminale déoxytransférase puis sont rassemblés avec un vecteur pBR322 à extrémité polyG coupé par Pst1. Les plasmides obtenus sont utilisés pour transformer des souches de E. coli (souche 1106) et les transformants tétracycline résistants (obtenus avec une efficacité comprise entre 500 et 1 000 par ng de cDNA double brin) sont mis en croissance sur des plaques de L-agar contenant 15 µg/ml de tétracycline. La banque de clone de cDNA représentant 43 000 transformants individuels est établie, les cellules sont récupérées par grattage à partir des colonies et remises en suspension dans un bouillon-L contenant la tétracycline. La suspension est effectuée dans le glycérol à 50 % et stockée à -20 °C.

Réalisation de la sonde d'ADN pour le criblage de la banque

Compte-tenu du fait que la séquence d'amino-acide publiée pour l'hirudine (figure 1) ne présente pas de méthionine ou de tryptophane, c'est-à-dire les amino-acides qui sont codés par un codon unique, la séquence de protéine ne présente pas de régions particulièrement propices pour la réalisation de sondes d'oligonucléotide particulièrement court qui constitue en règle générale la stratégie mise en oeuvre pour l'identification des séquences d'ADN cloné (24). C'est pourquoi il a été décidé d'adopter la technique différente d'une sonde d'oligonucléotide assez grande mais unique, ce qui a permis en particulier d'isoler le clone de cDNA pour le facteur IX de coagulation humain (23). Dans cette stratégie, on sélectionne la région de séquence d'amino-acide pour lequel la redondance des codons est limitée au choix de la 3ème base (c'est-à-dire que l'on écarte l'arginine, la leucine et la sérine), et on sélectionne la 3ème base. Les paramètres considérés pour choisir la 3ème base de ce codon, sont connus, il s'agit de maximiser la possibilité d'interaction G/T et d'écarter les épingles à cheveux et les palindromes dans la séquence de la sonde et bien entendu de tenir compte des séquences de gènes connues pour les sangsues.

Comme aucune séquence de gène n'a été publiée pour les sangsues on a dû faire usage des connaissances sur les êtres les plus proches des sangsues au plan de l'évolution et sur lesquels il existe de nombreuses séquences d'ADN publiées, à savoir les insectes. Toutes les séquences codantes pour des ADN d'insectes ont été analysées et une table des codons que l'on retrouve le plus fréquemment a été établie. Ceci avec d'autres paramètres a été utilisé pour concevoir un oligonucléotide de 48 bases de longueur, correspondant à 16 amino-acides, de la position 34 à la position 49 dans la séquence de l'hirudine. Cette région de séquence présente une redondance seulement dans la troisième position de chaque codon.

Cet oligonucléotide représenté à la figure 2 a été synthétisé chimiquement par la méthode au phosphodiester, sur un support solide inorganique (25), qui a déjà été décrit pour un 52 mer en référence (23).

Criblage de la banque de cDNA avec l'oligonucléotide 48 mer.

La banque de cDNA tenue à partir des mARN de têtes de sangsues a été étalée sur des plaques d'agar L contenant 15 µg/ml de tétracycline avec une densité de colonie d'environ 4 000 colonies par plaque de 13 cm et les colonies sont mises en croissance jusqu'à une taille de 1 à 2 mm de diamètre à 37 °C. Les colonies sont alors prélevées sur filtres de nitrocellulose. Les colonies des plaques maintenues sont mises en croissance puis les plaques sont stockées à 4 °C. Les filtres sont placés sur les plaques de L-agar contenant 170 µg/ml de chloramphénicol et sont incubés une nuit à 37 °C pour amplifier les plasmides dans les colonies bactériennes. Les filtres sont ensuite traités par la procédure standard pour lyser les colonies bactériennes et fixer l'ADN sur la nitrocellulose. Les filtres sont ensuite lavés très complètement puis préhybridés dans un volume de 100 ml de 6 x SSC (1 x SSC = 0,15 % NaCl, 0,015 M de citrate trisodique), 5 x Denhardts (1 x Denhardts solution = 0,02 % Ficoll, 0,02 % de polyvinyl pyrollidone, 0,02 % d'albumine de sérum bovin) 100 µg/ml d'ARN de transfert de levure et 0,05 % de pyrophosphate de sodium.

Un aliquote (30 pmole) de l'oligonucléotide 48 mer est marqué avec [$^{32}$P] à son extrémité 5' par incubation avec $\gamma$ [$^{32}$P] ATP en présence de 15 unités de polynucléotides kinase. La sonde marquée est purifiée du

$\gamma$ [$^{32}$P] ATP libre par passage sur cellulose DEAE. La sonde marquée est hybridée sur le filtre à la dose de 20 ml de 6 x SSC, 1 x Denhardts, 100 µg/ml de tRNA de levure, 0,05 % de pytophosphate de sodium à 42 °C sous agitation légère pendant 16 heures.

Les filtres sont alors lavés dans 6 x SSC, 0,1 % de dodécyl sulfate de sodium (SDS) à la température

de 37°C, 42°C, 50°C puis soumis à un lavage final avec 2 x SSC, 0,1 % SDS à 50°C pendant 10 minutes. Les filtres sont alors séchés et exposés sur des films rayons X. Les colonies donnant des résultats positifs sont repérées à partir des plaques marquées en comparant les spots positifs sur le film X avec les plaques maitresses. L'un de ces clones dont le plasmide sera nommé pTG700 est confirmé comme étant positif par une seconde hybridation avec la sonde marquée est cultivé en quantité et l'ADN plasmidique est purifié par des procédures standards.

Identification de pTG700 comme contenant la séquence codant pour l'hirudine.

L'insert de cADN de pTG700 représente environ 235 paires de bases. Ce fragment d'ADN est isolé et transféré dans le phage vecteur m13 mp8 et la séquence d'ADN de l'insert est déterminée par terminaison de chaîne (26). Une partie de cette séquence code pour une séquence protéinique qui est très similaire à celle de l'hirudine. Cette région de la séquence correspond à la zone de la sonde qui est représentée à la figure 2.

Sur cette figure 2 :

(a) correspond à la séquence de l'hirudine figurant dans la référence 2 de glu 49 à gly 34,
(b) correspond à la séquence de la sonde 48 mer synthétisée et utilisée pour l'hybridation,
(c) correspond à la séquence du clone pTG700 dans cette région, les points indiquant les homologies,
(d) correspond à la séquence d'amino-acide codée dans cette région de pTG700.

Il faut remarquer que la séquence de cADN dans pTG700 est incomplète, elle code seulement 28 des amino-acides C-terminaux de l'hirudine en plus des 101 base de la séquence non traduite 3' précédée par un codon stop en phase. L'une des différences par rapport à la séquence de protéine publiée pour l'hirudine est observée dans ce clone car l'acide glutamique remplace la glutamine à la position 49.

Isolement de clones de cADN d'hirudine plus grand.

Comme la séquence d'ADN confirme que l'insert de pTG700 code pour l'hirudine, cet insert est rendu radioactif par [$^{32}$P] par la procédure de "nick-translation" connue et est utilisée pour un nouveau test de la banque de cDNA. Plusieurs autres colonies positives sont trouvées et l'une d'entre elle comporte un plasmide désigné par pTG717 qui contient un insert d'une longueur d'environ 450 paires de bases. L'analyse de restriction de cet insert indique que la présence d'un site de restriction TaqI placé au voisinage du centre.

C'est pourquoi le fragment purifié est mis en digestion avec TaqI et les fragments résultant 5' et 3' sont liés dans le vecteur de séquensage m13 clivé par PstI/AccI. La construction du m13 contient chacun des fragments qui peuvent être identifiés et soumis à l'analyse séquentielle. Le résultat de cette analyse est indiqué à la figure 3. La séquence d'ADN de l'insert de pTG717 moins les extrémités polyG/C introduites à l'extrémité pendant le clonage de la procédure est long de 379 bases, dont 219 codent un peptide dont la séquence est très similaire à celle de la séquence de l'hirudine publiée.

Différents points doivent être notés dans cette séquence.

1) la séquence d'amico acide codée par le fragment n'est pas identique à celle de la séquence de la protéine publiée pour l'hirudine native. Il y a 9 amino-acides de différence entre la séquence de l'hirudine isolée à partir de pTG717 et la séquence de l'hirudine publiée. On note les modifications suivantes : Val 1 — ile ; val 2 — thr ; gln 24 — lys ; asp 33 — asn ; glu 35 — lys ; lys 36 — gly ; lys 47 — asn ; gln 49 — glu et asp 53 — asn. Ceci constitue un changement majeur dans la séquence car 7 de ces modifications impliquent un changement de charge. L'homologie entre la séquence codante clonée et la séquence de la protéine publiée est ainsi de 46/65, c'est-à-dire d'environ 70 %.

Il existe deux raisons possibles pour les différences observées entre les deux séquences d'amino-acides.

1° - On peut voir des espèces, sous-espèces et mêmes des variations animales individuelles entre la séquence exacte de la molécule d'hirudine. Il n'est pas possible de vérifier si les sangsues utilisées dans cette étude sont exactement de la même espèce ou sous-espèce que celles qui ont été utilisées pour publier la séquence d'amino-acide. En outre, il est possible qu'il existe dans les sangsues non seulement une mais différentes formes d'hirudine avec une activité biologique similaire mais avec des variations dans les séquences d'amino-acides de base. Dans ce contexte, il est significatif de mentionner les différences des résultats dans la littérature concernant les amino-acides N-terminaux de l'hirudine (à l'origine ile avait été trouvé dans la matière des têtes, puis val avait été trouvé dans l'ensemble de l'animal, tandis que plus récemment on a de nouveau indiqué ile pour la matière provenant des têtes. L'isoleucine se trouve dans la séquence de pTG717 à la position correspondant à l'extrémité N-terminal

de la protéine mature.

Le concept de différentes formes d'hirudine est supporté par les études préliminaires au niveau de l'ADN. Lorsque l'ADN total des sangsues est extrait à partir de sangsues congelées broyées par des procédés standards, digérées par divers enzymes de restriction traité par électrophorèse sur gel d'agarose, puis transférés sur nitrocellulose et hybridés avec l'insert pTG717 comme sonde, on observe une grande quantité de fragments qui s'hybrident.

Ainsi les résultats de la figure 4 sont obtenus de la façon suivante :

- 10 µg de l'ADN total de sangsues sont mis en digestion avec des enzymes de restriction et pressés en électrophorèse sur un gel d'agarose à 1 %, transférés sur des filtres de nitrocellulose et hybridés avec un insert PstI marqué au [ $^{32}$P] de pTG717. Les filtres sont alors lavés fortement (0,1 x SSC, 0,1 % SDS, 65 ° C) et les bandes hybridées sont visualisées par autoradiographie.

  Ligne 1 - ADN digéré avec EcoRI
  Ligne 2 - ADN digéré avec HindIII
  Ligne 3 - ADN digéré avec BamHI
  Ligne 4 - ADN digéré avec BglII

La dimension des fragments EcoRI en kb est indiquée à droite.

Avec les fragments de digestion Eco RI, 6 fragments totalisant 40 kb peuvent se voir dans des conditions de lavage très dures. Même s'il est théoriquement possible que ce schéma représente un gène mosaïque très large, il est peu probable qu'une seule petite séquence codant pour moins de 400 bases soit distribuée sur 40 kb de génome. En outre, des expériences préliminaires avec des sondes comportant des fragments à insert partiel suggèrent que cela n'est pas le cas.

Il a également été observé qu'il existait une différence dans les séquences dans différents clones d'hirudine, isolés à partir de la même banque de cADN. Par exemple dans pTG700 un lys se trouve en position 47 (figure 2) comme dans la séquence d'amino-acides publiée, tandis que dans pTG717 il existe un asn à cette position.

C'est pourquoi toutes ces informations sont consistantes avec le concept de gènes d'hirudine de structure différente et présentant des formes multiples au niveau de la protéine.

Un point finalement important est que la séquence entière du mARN d'hirudine n'est certainement pas contenue dans pTG 717. En effet, la lecture ouverte continue jusqu'à l'extrémité 5' du clone et il n'y a pas de méthionine dans cette région. Comme l'hirudine est une protéine qui est sécrétée à partir des cellules des glandes salivaires, on peut penser qu'elle a à son extrémité N-terminal une séquence "leader" (nécessaire pour la sécrétion) et probablement aussi une pro-séquence. La molécule active finale serait alors produite comme cela est le cas pour de nombreuses zymogènes et précurseurs par des étapes de clivage protéolytiques.

La taille du mARN de l'hirudine a été déterminée et confirme que le clone pTG717 n'en n'est pas une copie complète. L'ARN des têtes total d'hirudine a été passé sur électrophorèse sur un gel d'agarose formaldéhyde dénaturé (27) transféré sur nitrocellulose et hybridé avec un insert de pTG717. Une espèce d'ARN hybride unique de 640 paires de bases est observée. Comme on pense que le pTG717 contient la séquence de mARN 3' entière (une portion polyA est observée) de même que deux sites d'addition de polyA qui se recouvrent, environ 20 base à partir du polyA, on voit une région non traduite 3' de 160 bp (figure 3). Ce clone de cDNA manque de 160 paires de base à l'extrémité 5'. Il comprend le reste de l'extrémité N-terminal de la protéine, la méthionine de l'initiateur, et une région non traduite 5' du mARN. Il n'est pas exclu que l'hirudine soit coupée à partir de l'extrémité C sous forme d'un précurseur plus important, qui peut coder pour d'autres peptides actifs biologiquement, bien que la taille maximum de ce précurseur hypothétique serait au moins de 110 à 120 amino-acides, (c'est-à-dire environ le double de la taille de l'hirudine mature).

Il n'a pas été possible de générer les clones de cADN d'une longueur supérieure à celle de pTG717 et on pense qu'une structure secondaire dans l'extrémité 5' du mARN empêche la transcription inverse du mARN au delà de ce point. L'analyse de la séquence génomique de l'hirudine devrait fournir des informations sur l'extrémité 5' de ce gène.

La séquence d'amino-acides de l'hirudine codée par le clone pTG717 a été adaptée pour s'exprimer dans les cellules de E. coli de la façon qui sera décrite ci-après.

Expression de l'hirudine dans E. Coli

A partir de l'analyse de restriction de pTG717 (figure 5) il est clair que l'ensemble de la séquence codant de l'amino-acide est contenu sous forme d'un fragment de 225 bp HinfI/AhaIII. Ce fragment est isolé par restriction et inséré dans les vecteurs d'expression plasmidique pTG951 et pTG927 en utilisant des

molécules d'adaptateurs d'oligonucléotide synthétique. Les adaptateurs constituent les 7 amino-acides qui ont été éliminés de l'extrémité N-terminal par la restriction HinfI et on ajoute le fragment méthionine initiateur et le site pour les endonucléases de restriction NdeI ou BglII qui sont nécessaires pour l'insertion dans les vecteurs d'expression.

Les deux amino-acides après l'initiateur met sont remplacés, c'est-à-dire que le fragment ile-thr qui figure dans le clone de cDNA pTG717 est remplacé par val-val de la séquence de protéine d'hirudine publiée.

Bien que le clone de cADN pTG717 comporte la séquence ile-thr au lieu de la séquence val-val de la séquence publiée pour l'hirudine, il a été décidé initialement d'exprimer la molécule avec une séquence val-val à l'extrémité N-terminal. Il y a deux raisons à ce choix :

- le clone de cADN est certainement incomplet, avec probablement une structure secondaire dans le mARN qui empêche la transcription. Ceci rend possible que l'extrémité 5' de la séquence de cADN soit incorrecte compte-tenu de la transcription et du clonage "d'artéfacts",
- en général on admet que l'extrémité N-terminal de la molécule d'hirudine active extraite de l'animal complet commence avec val-val (8, 11).

Le vecteur d'expression pTG951 est un vecteur d'expression plasmidique destiné à exprimer le gène de l'interféron $\gamma$ dont la synthèse est rappelée à la fin de la présente description.

Pratiquement, il contient essentiellement le promoteur gauche du bactériophage $P_L$ contrôlé par un répresseur codé par l'hôte thermosensible C1857, suivi par le gène N de $\lambda$ , intact ou tronqué. Ensuite on trouve un site de fixation des ribosomes synthétiques qui a été conçu pour donner une fixation des ribosomes optimale. Un site de restriction unique BglII se trouve situé entre le site de fixation des ribosomes et l'ATG de la séquence codant pour l'interféron $\gamma$. Ce vecteur est mis en digestion sur le site BglII et au site unique PvuI en aval de la séquence codant pour l'interféron $\gamma$ puis est récupéré par électrophorèse sur gel d'agarose. Ce fragment de vecteur est combiné avec un ensemble d'oligonucléotide d'adaptateurs et la séquence d'hirudine contenant le fragment HinfI/AhaIII tel que cela est décrit dans la figure 6.

Le vecteur d'expression pTG927 est étroitement relié au vecteur d'expression pTG908 décrit

à la fin de la présente description. Il diffère seulement en ce qu'il contient un fragment additionnel SalI-PvuII provenant du gène de la tétracycline de pBR322. Le vecteur d'expression pTG927 contient le promoteur $P_L$, un gène N intact et le site de fixation des ribosomes de la protéine CII de $\lambda$ puis l'ATG et une séquence codant pour le fragment lacZ de la $\beta$-galactosidase. Sur l'ATG de cette séquence codante on trouve un site NdeI. Ce vecteur est mis en digestion avec NdeI et PvuII et le fragment de vecteur est purifié. Il est utilisé ensemble avec un autre jeu d'adaptateur d'oligonucléotide et le fragment HinfI/AhaIII de l'hirudine pour construire le second vecteur d'expression de l'hirudine qui figure à la figure 6. Les deux vecteurs d'expression sont destinés à exprimer la molécule d'hirudine native (avec une séquence val-val à l'extrémité N-terminal immédiatement après l'initiateur ATG).

Dans l'oligonucléotide adaptateur le codon de la 3ème base est choisi pour favoriser un haut degré de traduction et pour éviter la formation de structures secondaires dans les mARN au niveau de cette région.

Les deux vecteurs sont assemblés de la façon suivante : les oligonucléotides sont phosphorylés à l'extrémité 5' par la polynucléotide kinase, puis traités en mélange équimolaire (après 10 minutes de chauffage à 65 °C pour 15 heures à 15 °C). Le vecteur et le fragment d'hirudine sont ajouter en quantité destinés à donner des rapports molaires du vecteur /les fragments d'hirudine/fragment d'adaptateur de 1:20:50 et le mélange est lié avec la ligase ADNT4. Le mélange de ligation est utilisé pour transformer la souche E. coli TG900 et les transformants portant les plasmides sont sélectionnés sur la plaque d'agar contenant de l'ampicilline (100 $\mu$g/ml) et ceux qui comportent la séquence codant pour l'hirudine sont identifiés par hybridation des colonies en utilisant un insert marqué de pTG717 comme sonde. A partir d'un grand nombre de clones positifs, 6 de chaque, en même temps que 1 négatif (souche parente) sont sélectionnés et mis en croissance dans un milieu liquide de bouillon-L à 30 °C jusqu'à une densité optique de 0,3 à 650 nm. L'expression à partir du promoteur $P_L$ est ensuite induite par une augmentation de la température à 37 °C et une incubation qui est maintenue pendant 6 heures. Les cellules sont alors récoltées par centrifugation, suspendues 1/5 du volume de TGE (25 mM Tris HCl, pH 8, 50 mM glucose, 10 mM EDTA) puis broyées par sonification. Après clarification des extraits par centrifugation, un aliquote du surnageant est testé pour son activité antithrombinique. Des niveaux significatifs d'activité antithrombinique apparaissent dans 5 des 6 constructions avec pTG 927 et dans 4 des constructions sur 6 avec pTG 951 qui indiquent également une activité bien qu'elle soit plus faible.

Les contrôles n'indiquent aucune activité significative. L'analyse de la séquence d'ADN des clones donnant des résultats positifs par séquençage direct des plasmides montrent que la séquence attendue se trouve dans toutes les constructions qui présentent une activité.

Deux clones présentant une expression positive, le clone pTG719 dérivant de pTG951 et le clone pTG718 dérivant de pTG917 sont analysés pendant 6 heures d'induction. Après croissance de la culture à 30° jusqu'à une densité optique de 0,3, l'expression est induite par une augmentation de la température à 37°C et un aliquote est prélevé toutes les heures pendant 6 heures des extraits tels que préparés précédemment et l'activité en hirudine est mesurée (29). Les résultats (figure 7) montrent que le clone pTG719 présente une croissance initiale suivie par un plateau de l'activité après 3 à 4 heures qui est au niveau de 370 U/l à une densité optique de 650.

Au contraire, le clone pTG718 présente une activité supérieure qui continue à croître pendant toute cette période d'induction.

Le niveau d'activité obtenu après 6 heures est environ 5 fois supérieur à celui du clone pTG719 et représente une activité totale de 7300 µ/l de culture. Si ce recombinant d'hirudine présente une activité spécifique similaire à celle du produit naturel val-val le produit obtenu dans les extraits les plus actifs correspondraient à environ 1 mg/l de culture.

Propriété de l'hirudine obtenue.

Afin de caractériser l'hirudine obtenue à partir de E. coli on a analysé directement des échantillons de culture après induction, qui ont été chauffés à 70°C pendant 15 mn ou chauffés à 70°C après réduction du pH 2,8 avec HCl. Dans ce dernier casle pH de l'extrait à été ramené à la neutralité avant d'être dosé. L'hirudine native traitée de la même façon ne présente pas de perte d'activité, comme cela pouvait être attendu compte-tenu des propriétés publiées de la molécule (20). Dans le cas de l'extrait, aucune perte d'activité n'apparaît après ce traitement, en fait, après le traitement on observe une augmentation de l'activité d'environ 2 fois. Ceci peut refléter soit la dégradation, soit l'inactivation des constituants de l'extrait qui inhibaient l'action de l'hirudine, ou peut être le pH assez bas et l'étape de chauffage peuvent dans certains cas assurer une reformation plus complète des ponts disulfure qui sont nécessaires pour que l'hirudine présente une activité maximum. Les extraits de contrôle ne présentent aucune activité avant ou après le traitement.

Lorsque les extraits bactériens sont préincubés avec de la thrombine fixée sur une résine sépharose et que la thombine-sépharose est éliminée par centrifugation, pratiquement toute l'activité hirudine initiale se trouve éliminée de l'extrait. L'hirudine obtenue selon la présente invention semble se lier de façon très efficace à la résine thrombine-sépharose ce qui permet d'envisager une possibilité pour la purification de cette hirudine bactérienne.

Des cultures bactériennes de cellules contenant le vecteur d'expression pTG718 sont mises en culture jusqu'à une densité optique de 0,3 à 30°C puis induites à 37°C et des aliquotes sont prélevés toutes les heures et marqués, sur milieu minima avec de la méthionine $35_S$ 100 µCi/ml. Les bactéries sont rassemblées par centrifugation et l'ensemble des protéines bactériennes marquées sont analysées par électrophorèse sur gel de polyacrylamide SDS, suivies par une fluorographie.

Au moins deux polypeptides induits en quantité significative par la construction du vecteur peuvent être observés à environ 6 à 8 000 Daltons (figure 8 - lignes 5 à 10).

Lorsque les matériaux marqués à partir de cultures non induites de pTG718 et à partir de cultures de 3 et 5 heures après induction sont traités à 70°C à pH 2,8 pendant 15 mn, centrifugés pour éliminer les protéines dénaturées et analysées sur un gel de polyacrylamide SDS, il est clair que l'essentiel des protéines de E. coli marquées sont éliminées de l'échantillon (figure 9 - lignes 1 et 2). Cette procédure donne une purification très satisfaisante des 2 bandes de poids moléculaire faible qui sont induites dans les vecteurs d'expression de l'hirudine.

L'ensemble de l'activité hirudine se retrouve dans le surnageant.

Préparation des plasmides vecteurs pTG951 et pTG927.

Ces plasmides vecteurs ont été utilisés pour cloner l'interféron γ ou dérivés de plasmides entrant dans la préparation de tels vecteurs. C'est cette synthèse qui sera rappelée brièvement ci-après en regard des figures ci-annexées sur lesquelles :

- la figure 10 représente la préparation de pTG907
- la figure 11 représente la préparation de M13 TG910
- la figure 12 représente la préparation de pTG908
- la figure 13 représente la préparation de pTG909
- la figure 14 représente la préparation de pTG941
- la figure 15 représente la préparation de pTG951.

La préparation de ces plasmides vecteurs comporte essentiellement :
a - la préparation de pTG908 vecteur qui comporte $P_L$,N et CII rbs
b - la préparation de pTG951 vecteur qui comporte un site de fixation des ribosomes synthétiques.

Préparation de pTG907 (figure 10).

Le plasmide de base utilisé est le plasmide pBR322 ; toutefois, celui-ci présente l'inconvénient d'avoir à l'intérieur du gène $amp^R$ un site de restriction PstI, car un site de même nature sera utilisé par la suite dans la zone de clonage comme site unique de restriction. Il convient donc de faire disparaître ce site de restriction PstI en utilisant un mutant du plasmide pBR322, le le plasmide pUC8, dans lequel le gène de résistance à l'ampicilline ne présente pas de site de restriction PstI (ce site a été éliminé par mutation in vitro). pBR322 est commercialisé notamment par Bethesda Research Laboratories et pUC8 est décrit dans l'article référencé 30.

Pour ce faire, on échange le fragment PvuI/PvuII de 1 669 bp de pBR322 avec le fragment analogue PvuI/PvuII du plasmide pUC8. Afin de réaliser cet échange les plasmides pBR322 et pUC8 sont traités successivement par PvuI, PvuII, puis circularisés par action d'une ligase.

On obtient ainsi le plasmide pTG902 qui ne présente plus de site de restriction PstI et qui a également perdu le site de restriction NdeI présent à l'origine sur pBR322 (non représenté sur la figure). En outre, le plasmide pTG902 porte un fragment de 50 kb correspondant à la séquence laci' dans lequel se trouve le site PvuII.

Le promoteur $P_L$ et le gène λN (qui provient du phage λ, le gène λN code pour une fonction d'antiterminaison de transcription) sont isolés du plasmide pKC30 pour être insérés dans pTG902 comme celà est indiqué sur la figure 10 ci-annexée par traitement EcoRI, SI, BamHI pour pTG902 et par traitement PvuI, SI, BamHI pour pKC30 avec ligation.

L'un des plasmides obtenus après transformation de la souche TGE900, pTG906, est traité de façon à éliminer le segment PvuII-SalI. Pour ce faire, pTG906 est traité successivement avec SalI, la nucléase SI, PvuII et la ligase. On obtient ainsi pTG907.

Préparation de M13 TG910 (figure 11).

On insère ensuite la "région de fixation des ribosomes" λcIIrbs (provenant elle aussi du phage λ) sous forme d'un fragment AvaI/TaqI dans le début du gène LacZ' (fragment $\alpha$ de la $\beta$-galactosidase) lequel a été cloné dans le phage M13 nommé M13tg110. Cette stratégie permet un test fonctionnel simple pour rbs, à savoir la production de la protéine lacZ' et, par conséquent, d'obtenir des plaques bleues en présence d'IPTG et de Xgal ; ceci permet également un séquençage rapide de la construction en utilisant la méthode dite du didéoxy.

On obtient ainsi après sélection dans les bactéries compétentes un clone résultant M13tg910 dont la structure globale est représentée à la partie inférieure de la figure.

Préparation de pTG908 (figure 12).

Le fragment cIIrbs/lacZ' du phage M13tg910 est transféré sur le plasmide vecteur pTG907 préparé précédemment.

Pour ce faire, on élimine les sites EcoRI, BamHI et AvaI en amont de cIIrbs puis on insère un site BglII.

Dans ces conditions, cIIrbs peut être prélevé sous forme d'un fragment BglII-BglII et placé dans le site BamHI en aval du promoteur $P_L$ et du gène λN de pTG907.

Le phage M13tg910 est digéré avec EcoRI puis traité avec Bal31 puis ensuite par la polymérase de Klenow. Les fragments obtenus sont alors soumis à l'action de la ligase en présence d'adaptateur BglII non phosphorylés. Le mélange de ligation obtenu est utilisé pour transformer des cellules compétentes JM103.

On sélectionne alors les plages bleues. Ces clones sont ensuite analysés afin de vérifier qu'ils contiennent le site BglII et qu'ils ne présentent plus de site EcoRI ou BamHI en amont. On obtient ainsi des clones tels que M13tg 912 dont la structure est représentée.

Le traitement par Bal31 a produit une délétion de 101 bp éliminant les sites EcoRI, BamHI et AvaI ; ainsi que les séquences de lac ATG et lac Shine/Dalgarno. Le site BglII introduit se trouve placé environ 100 bp en amont de l'ATG de cII et 10 bp en aval de $P_{lac}$.

Le fragment BamHI/SphI de pTG907, le fragment BglII/HpaI portant cIIrbs et lacZ' et l'adaptateur phosphorylé ont été préhybridés dans un rapport molaire de 1:2:1 puis traités avec la ligase $T_4$. Des aliquots sont utilisés pour transformer les cellules compétentes de la souche 6150 à 3 °C.

Les cellules intéressantes sont identifiées en sélectionnant les transformants avec un fragment cIIrbs/lacZ' marqué au $P^{32}$ et la construction obtenue est confirmée par une étude de restriction enzymatique.

Afin d'avoir une première indication montrant que les différents éléments du système d'expression se conduisent comme cela est désiré, le plasmide obtenu, pTG908, est trapsféré dans une souche hôte N6437 qui possède à la fois c1857 et le fragment $\omega$ de la $\beta$-galactosidase complémentant le fragment $\alpha$ qui est codé par le plasmide.

Les transformants obtenus placés sur une boite contenant IPTG + Xgal sont blancs à 28° C puis virent au bleu environ 30 minutes après lorsqu'on les transfère à 42° C.

Ce vecteur avant d'être utilisé pour cloner l'hirudine a été adapté pour cloner l'interféron $\gamma$ humain : IFN-$\gamma$, en fait pour le clonage de l'hirudine la nature de la protéine intermédiaire clonée n'a pas d'importance, mais les vecteurs ont été réalisés selon le schéma ci-après.

L'analyse de la séquence des nucléotides de IFN-$\gamma$ pour les sites de restriction révèle un site EcoRII à 8 bp en aval du départ de la protéine mature et un site Sau3A à 285 bp en aval du codon stop, ce qui permet d'isoler pratiquement toute la séquence codant pour la protéine mature sur un fragment EcoRII/Sau3A. Le clone de IFN-$\gamma$ obtenu à partir d'une banque est nommé pTG11.

Construction de pTG909 (figure 13).

On utilise tout d'abord une molécule d'adaptation synthétique qui permet :

a) d'effectuer la jonction entre les extrémités EcoRII et NdeI,

b) d'introduire les 8 bp manquantes par rapport à la séquence codant pour IFN-$\gamma$ mature et,

c) de reconstituer le codon de départ ATG de cIIrbs de façon que la séquence codant pour la protéine IFN-$\gamma$ mature soit traduite sans amino-acides fusionnés à l'exception de l'initiateur F-met.

Cet adaptateur est synthétisé chimiquement et sa constitution est représentée sur la figure.

pTGII est mis en digestion avec EcoRII et Sau3A et pTG908 avec NdeI et BamHI.

Les fragments appropriés sont purifiés sur gel, mélangés avec une quantité équimolaire de l'adaptateur, préhybridés et ligés. Le mélange est utilisé pour transformer des cellules compétentes TGE900 et les transformants sont sélectionnés en hybridant un insert PstI de pTGII "nick-traduit" et marqué au $P^{32}$ avec les transformants.

13 clones sont sélectionnés et contrôlés par cartographie et l'un d'eux pTG909 est vérifié par séquençage.

Construction du vecteur pTG941 (figure 14).

pTG909 contient 2 sites NdeI, l'un au codon de départ de IFN-$\gamma$ et l'autre 22 bp eb aval de la séquence IFN-$\gamma$.

La région entre ces sites qui est la région codant pour les 7 premiers amino-acides de IFN-$\gamma$ a été éliminée par traitement avec NdeI et remplacée par un oligonucléotide synthétique qui est représenté sur la figure.

Cette réaction détruit le site NdeI aval et reconstitue le site NdeI amont tout en introduisant un site BamHI qui est unique. On obtient ainsi le vecteur pTG941.

Construction de PTG951 (figure 15).

La figure 15 schématise la construction de pTG951 qui est dérivé de pTG941 dans lequel le fragment contenant le cIIrbs a été remplacé par une séquence synthétique sur la base de la séquence de la région d'initiation de la traduction de l'opéron E. coli lac noté E. coli lac opéron rbs. Cet oligonucléotide synthétique a été inséré au niveau du site HgaI entre le site unique NdeI du codon de départ de la séquence codant pour IFN-$\gamma$ et le site ClaI qui a été inséré dans le gène N.

De ce fait par traitement avec NdeI et ClaI le plasmide pTG951 ne contient plus qu'un gène N tronqué (un codon stop en phase avec la traduction du gène N est placé immédiatement en amont du nouveau site rbs) et est dépourvu des terminateurs de transcription tLI et tRI présents dans pTG909 et pTG941.

Les principaux résultats sont rappelés dans le tableau ci-après :

| NOM | PROMOTEUR | RBS | SEQUENCE DE RBS ET JONCTION AVEC LA SEQUENCE |
|---|---|---|---|
| | | | fmet cys tyr cys gln asp pro |
| pTG909 | PL | cII | TAAGGAAGTACTTACATATG TGT TAC TGC CAG GAC CCA |
| | | | fmet cys tyr cys gln asp pro |
| pTG941 | PL | cII | TAAGGAAGTACTTACATATG TGC TAC TGT CAG GAT CCC |
| | | | fmet cys tyr cys gln asp pro |
| pTG951 | PL | SYNTH (lac) | CACAGGAACAGAGATCTATG TGC TAC TGT CAG GAT CCC<br>------<br>BglII |

Les exemples ci-après sont destinés à illustrer la préparation du variant HV2 modifié à son extrémité N-terminale.

Sur les figures annexées :

- la figure 16 représente une courbe de l'activité hirudine induite dans une culture de E. coli TG900 contenant pTG720 ;
- la figure 17 représente un spectre d'analyse des protéines marquées au $^{35}S$ des extraits de E. coli TG900 contenant pTG720.

Préparation de HV2 modifié

La construction du plasmide pTG720 exprimant l'hirudine selon l'invention est obtenue selon le même processus que le plasmide pTG718 décrit précédemment en partant de pTG717 et de pTG927.

On assemble le fragment NdeI/PvuII de pTG927 avec le fragment HinfI/AhaIII de pTG717 par l'intermédiaire d'oligonucléotides adaptateurs,décrits ci-après,afin de reconstituer à l'extrémité N terminale de l'hirudine la séquence ile - thr.

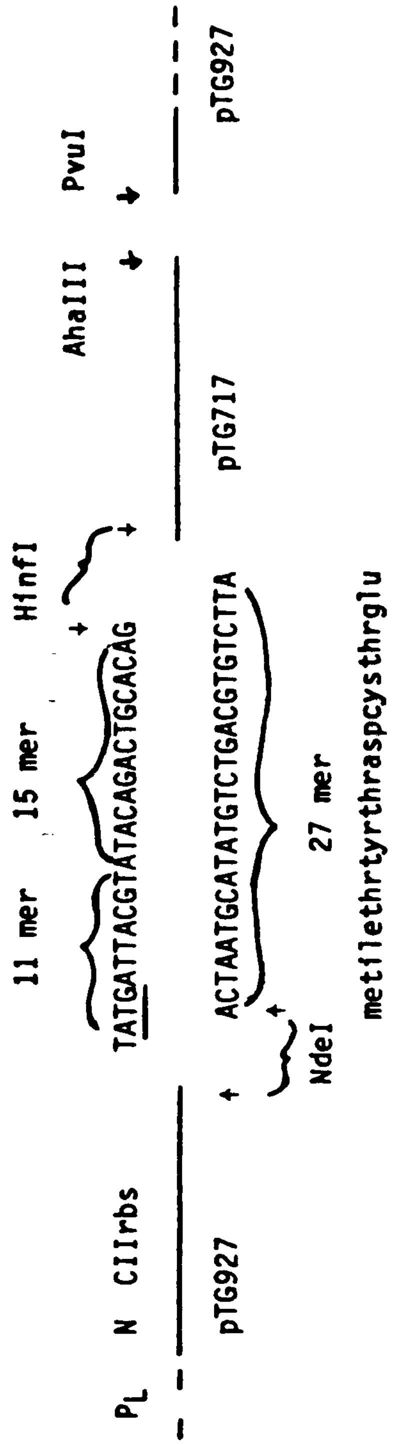


Le mélange de ligation est utilisé pour transformer E. coli TG900 et les transformants contenant des plasmides sont sélectionnés sur plaque de L agar contenant 100 µg/ml d'ampicilline. Les constructions qui contiennent la séquence de l'hirudine sont identifiées par hybridation des colonies en utilisant un insert marqué de pTG717 comme sonde.

La séquence d'ADN du plasmide final a été contrôlée par séquençage direct de l'ADN dans le plasmide d'expression.

Expression de l'activité hirudine par pTG720

Des cellules de E. coli TG900 contenant le plasmide pTG720 sont mises en croissance sur milieu LB plus 50 µg/ml d'ampicilline à 30 ° C jusqu'à une densité optique 600 de 0,3.

Les cultures cellulaires sont alors transférées à 37 ° C pour induire la transcription à partir du promoteur $P_L$.

On prélève heure par heure 1 ml d'une aliquote et la densité à 600 nm est mesurée, puis les cellules sont collectées par centrifugation.

Le culot de centrifugation est remis en suspension dans 200 µl de TGE (25 mM Tris HCl, pH 8,0, 50 mM glucose, 10 mM EDA) et les cellules sont lysées par sonication.

Après clarification, le surnageant est collecté et l'activité antithrombine est mesurée, soit par l'essai de coagulation ou par dosage colorimétrique de l'inhibition de la coupure du substrat, tosyl-glycil-prolyl-arginine-4 nitroanilide acétate (Chromozym TH, Boehringer Mannheim GmbH) par une solution de thrombine standard.

La réaction est conduite dans un volume réactionnel de 1 ml en utilisant 13 µM de substrat dans un tampon composé de 100 mM Tris HCl, pH 8,0, 0,15 M KCl, 0,1 % de polyéthylène glycol 6000.

La réaction avec 0,25 U de thrombine est suivie pendant 2 minutes grâce à un spectrophotomètre à 405 nm et la vitesse de réaction est mesurée à partir de la pente d'accroissement de densité optique.

De l'hirudine standard ou des extraits inconnus sont ajoutés à ce mélange réactionnel de thrombine pour déterminer la quantité d'inhibition ou l'activité antithrombinique.

La figure 16ci-annexée montre l'effet de l'induction de l'activité antithrombinique d'une culture de cellules contenant pTG720 exprimé en unité antithrombine pour une densité optique de 600 par litre de culture et ceci sur une période de 6 heures.

La ligne pointillée indique la courbe de croissance des cellules de E. coli mesurée à une densité optique de 600 pendant la même période.

On observe une activité de type hirudine présentant un niveau significatif grâce à l'induction.

Des lysats de contrôle des cultures contenant le plasmide sans la séquence hirudine ne présentent aucune activité.

Lorsque ces lysats bactériens sont chauffés à 70 ° C pendant 15 minutes après acidification à pH 2,8 avec HCl, une quantité considérable de protéines est dénaturée et précipitée. Lorsque celle-ci est éliminée par centrifugation de l'extrait refroidi et le surnageant neutralisé par addition d'un tampon Tris HCl (concentration finale 100 mM, pH 8,0) au moins 100 % et souvent plus de l'activité de départ se retrouve dans le surnageant.

Dans une expérience typique, 130 % de l'activité de départ se retrouve dans le surnageant.

Aucune activité résiduelle n'est trouvée dans le matériel précipité dans le culot.

Lorsqu'un extrait bactérien, chauffé et acidifié après refroidissement, centrifugation et neutralisation (200 µl contenant 5 ATUs d'hirudine) est incubé pendant 15 minutes à 37 ° C avec 100 µl d'une bouillie à 50 % de thrombine couplée de façon covalente à une résine Sepharose (préparée par des procédures standards) et lorsque la Sepharose thrombine est éliminée par centrifugation, aucune activité de type hirudine ne peut se trouver dans le surnageant.

Ainsi, l'hirudine produite par pTG720 a les mêmes propriétés générales que la molécule native et que celle qui est obtenue par pTG717 et pTG718.

Les polypeptides spécifiquement induits dans la culture de pTG720, après marquage avec la méthionine $S^{35}$, résolution par électrophorèse sur gel de polyacrylamide et visualisation par fluorographie, sont représentés à la figure 2. Une série de polypeptides de petit poids moléculaire (5 à 10 000 daltons) sont plus particulièrement induits.

Sur la figure 17,

- la ligne 1 représente les cellules non induites,
- la ligne 2 l'induction à 0 heure,
- la ligne 3, 1 heure d'induction,
- la ligne 4, 2 heures d'induction,
- la ligne 5 : marqueurs de poids moléculaire,
- La ligne 6, 3 heures d'induction,
- la ligne 7, 4 heures d'induction,
- la ligne 8, 5 heures d'induction,
- la ligne 9, 6 heures d'induction,
- la ligne 10, 7 heures d'induction.

Les exemples suivants sont destinés à illustrer la préparation de l'hirudine HV1.

Etude du gène HV1 et stratégie de synthèse

La stratégie de synthèse pour la préparation du gène de l'hirudine HV1 comprend différentes étapes.

Tout d'abord, comme il n'y a aucune différence en amino-acides entre HV1 et HV2 après l'amino-acide 53, comme cela ressort de la figure 18, et comme il y a un site unique TaqI dans le cADN de HV2 cloné dans pTG717 qui est centré sur l'amino-acide 56, (figure19), la séquence d'ADN du variant HV1 après l'amino-acide 56 peut être fournie par le fragment TaqI-PstI de pTG717.

C'est pourquoi seul l'ADN codant pour les 56 premiers amino-acides de HV1 doit être synthétisé chimiquement.

Cet ADN a été synthétisé en deux blocs séparés. Le premier, représenté sur la figure 20 débute par un site cohésif EcoRI qui est essentiellement destiné au clonage, immédiatement suivi par un site NdeI qui incorpore le codon d'initiation ATG avant la séquence codant pour HV1. Le gène complet peut être prélevé en utilisant le site NdeI à l'extrémité 5' pour l'insertion dans un vecteur d'expression dans E. coli. Cette partie est suivie par une extension d'ADN codant pour les amino-acides 1 à 32 de HV1 et peut être terminée par une extrémité cohésive BamHI, car les amino-acides 31 et 32 sont gly et ser et peuvent être codés par un site BamHI comme cela est représenté :

```
BamHI
↓
GGATCC

gly ser
```

Cette partie d'ADN synthétique qui présente 109 bp est assemblée par condensation de ses oligonucléotides constituants puis placée par ligation dans le phage M13mp8 coupé par EcoRI/BamHI de façon à conduire à la construction M13TG724. Comme l'ADN synthétique est contenu dans la région polylinker du phage M13, il peut être immédiatement séquencé pour vérifier l'assemblage correct de ce premier bloc synthétique.

Le second bloc synthétique correspond aux amino-acides 33 à 56 et est limité à une extrémité par un site cohésif BamHI et à l'autre extrémité par un site TaqI (figure 21). Ce bloc synthétique de 69 bp est de nouveau assemblé à partir de ses constituants oligonucléotides puis incorporé avec le fragment TaqI/PstI provenant de pTG717 dans M13TG724 coupé par BamHI/PstI comme cela est indiqué à la figure 21. Ceci conduit à un phage M13TG725 qui contient la séquence codant pour HV1 en entier. Comme précédemment, l'assemblage correct de cette construction peut être immédiatement vérifié par séquençage.

L'étape suivante comporte le transfert du fragment NdeI/AhaIII qui commence avec l'ATG de la séquence de l'hirudine et se termine dans une région non traduite à l'extrémité 3' dans le plasmide pTG927 coupé par NdeI/PvuII. Ce vecteur d'expression est identique à celui qui a été utilisé pour construire le vecteur d'expression hirudine HV2 pTG720 et sa structure et sa construction ont déjà été décrites. Le vecteur d'expression final codant pour le variant HV1 est appelé pTG726 et est représenté à la figure 22.

La séquence exacte des oligonucléotides utilisés pour la construction de ces deux blocs est indiquée à la figure 23. Le bloc 1 s'étend du site EcoRI au site BamHI et est composé de 8 oligonucléotides ayant des tailles de 22 à 32 bases. Le bloc 2 s'étend entre le site BamHI et le site TaqI et est composé de 6 oligonucléotides ayant des tailles s'échelonnant entre 19 et 30 bases.

Les oligonucléotides sont synthétisés par une procédure au phosphotriester manuelle sur un support de silice (référence 31) et sont purifiés en utilisant des techniques HPLC ou l'élution à partir de gel de polyacrylamide.

La séquence exacte des oligonucléotides utilisés pour la partie synthétique du gène est choisie en considérant les paramètres suivants :

a) le choix des codons, lorsque cela est possible, est celui des gènes qui sont exprimés à des très hauts niveaux dans E. coli ; ce choix est fait en utilisant les données publiées (réf.: 32, 33) ;

b) l'analyse par ordinateur de chacun des oligonucléotides individuellement, et ensuite dans la séquence complète, de façon à éliminer des structures pouvant former des "épingles à cheveux" ;

c) le choix de l'extrémité N-terminale de la molécule d'hirudine correspond à l'utilisation préférentielle de certains codons pour obtenir des bases dans certaines positions de cette région dont on a montré qu'elles étaient importantes pour une expression élevée de protéines étrangères dans E. coli.

Assemblage du gène synthétique

Premier bloc synthétique

Les oligonucléotides constituant ce bloc, 1-8, (figure 23) sont phosphorylés à leur extrémité 5' avec la polynucléotide kinase dans des conditions standards, sauf pour les deux oligonucléotides des extrêmes 1 et 8. Ceci est destiné à éviter la formation de dimère ou de polymère du bloc synthétique dans les étapes suivantes de ligation. 500 picomoles de chacun des oligonucléotides sont kinatées en utilisant 2 unités de polynucléotide kinase dans un volume final de 25 µl de 60 mM TrisHCl, pH 7,5, 10 mM $MgCl_2$, 8 mM dithiothréitol, contenant également 3,3 pmoles de $^{32}P$ γ-ATP, dont l'activité spécifique est de 5000 Ci/mmole. Après incubation pendant 15 minutes à 37°C, les oligonucléotides sont ensuite phosphorylés complètement par addition de 5 mmoles d'ATP froid.

Après incubation pendant 15 minutes supplémentaires à 37°C, les oligonucléotides sont purifiés par électrophorèse sur gel de polyacrylamide à 20 % conduite dans des conditions dénaturantes. Les oligonucléotides marqués sont détectés par autoradiographie, les régions appropriées du gel sont excisées et les oligonucléotides élués par $H_2O$ durant une incubation d'une nuit à 37°C.

Les oligonucléotides sont alors chargés sur des colonnes de DEAE-cellulose, élués par un tampon bicarbonate de triéthylammonium 1 M pH 8 et lyophilisés.

Pour les oligonucléotides 1 et 8 non kinatés et non marqués, la purification de gel est conduite comme précédemment mais les oligonucléotides sont détectés par absorption UV.

Les fragments complémentaires (1+5, 2+6 ...) sont mélangés, en utilisant des quantités équivalentes, 100 picomoles de chacun des oligonucléotides dans un volume final de 50 µl de 66 mM Tris HCl pH 7,5, 6 mM $MgCl_2$, 100 mM NaCl, 0,5 mM Spermidine, 8 mM dithiothréitol. Ces mélanges sont chauffés à 100°C et refroidis lentement à 37°C pendant 2 heures. Les solutions sont mélangées pour donner des hybrides avec 4 oligonucléotides dans 100 µl. Les mélanges sont finalement rassemblés et les 8 oligonucléotides sont mis à apparier une nuit à 37°C dans un volume final de 200 µl. 0,005 picomoles des oligonucléotides appariés sont ligués avec 25 ng de M13mp9 digéré par BamHI/EcoRI et purifié sur gel,

dans un volume final de 20 µl d'un mélange de ligation contenant 66 mM Tris pH 7,5, 6,6 mM $MgCl_2$, 10 mM dithiothréitol, 0,5 mM ATP.

La ligation se poursuit à 15°C pendant 24 heures, suivie de nouveau par 24 heures à 4°C. Le mélange de ligation est alors utilisé pour transformer E. coli JM103. A partir des nombreuses plaques blanches obtenues par transformation, on sélectionne 8 candidats, on prépare les ADN des phages monobrin et on les soumet à un séquençage d'ADN direct par la méthode de termination de chaîne didéoxy (réf. 34). De ces candidats, deux sont trouvés contenir l'assemblage correct d'oligonucléotides correspondant au bloc 1 de la figure 23 et l'un d'entre eux est désigné comme étant M13TG724 et utilisé dans l'étape suivante.

Second bloc synthétique et assemblage de l'ensemble du gène

Pour assembler le second bloc synthétique, on utilise essentiellement la même stratégie que celle qui a été décrite précédemment, à l'exception du fait que les constituants oligonucléotides ne sont pas prépurifiés avant l'étape d'appariement mais sont plutôt kinatés (sauf pour les oligonucléotides terminaux 9 et 14 - figure 23) puis directement appariés . Les conditions de d'appariement sont les mêmes que celles qui ont été décrites précédemment avec 100 picomoles de chacun des oligonucléotides dans un volume final de 150 µl.

Après l'étape d'appariement , le mélange est chargé sur un gel d'agarose à 2 % (agarose à basse température de fusion) puis passé en électrophorèse. Les oligonucléotides sont détectés par coloration au bromure d'éthydium et les bandes correspondant au bloc assemblé (69 bp) sont coupées du gel et éluées par des procédures standards.

Le second bloc synthétique (2 ng) est ensuite mélangé avec 50 ng du phage M13TG724 coupé par PstI/BamHI et purifié sur gel et 2 ng d'un fragment TaqI/PstI de pTG717 purifié sur gel. L'ensemble de ces éléments est assemblé dans un volume de 20 µl de 66 mM Tris HCl pH 7,5, 6,6 mM $MgCl_2$,chauffé à 65°C pendant 5 minutes, et on ajoute DTT jusqu'à 10 mM, ATP jusqu'à 0,5 mM et 5 unités de ligase $T_4$. La ligation est poursuivie pendant 16 heures à 15°C puis le mélange de ligation est utilisé pour transformer E. coli JM103.

A partir des plaques blanches parmi les transformants, on en choisit 12 qui sont sélectionnées pour préparer un phage monobrin pour séquençage direct. En outre, le phage est également préparé sous forme double brin (réf.35) pour étudier l'existence d'un site unique BamHI qui doit être présent dans les recombinants correctement assemblés. La majorité de ces clones contiennent à la fois le site BamHI et la séquence d'ADN correspondant à l'assemblage correct de l'ensemble du gène codant pour HV1. On choisit l'un de ceux là dénommé M13TG725.

Transfert du gène HV1 dans le vecteur d'expression

L'étape finale pour créer le plasmide vecteur capable d'exprimer la protéine HV1 consiste à transférer le segment de 248 bp NdeI/AhaIII de M13TG725 dans pTG927 coupé par NdeI/PvuII (figure 22). Toutefois, comme la forme réplicative du phage avec ce type de digestion conduit à un second fragment de M13 ayant pratiquement la même taille mais qui se clone de façon plus efficace dans le vecteur d'expression que le fragment désiré, il a été nécessaire tout d'abord de préparer un fragment AvaII/BglII (1,71 kb) qui contient l'ensemble de la séquence hirudine HV1 puis de le digérer avec NdeI/AhaIII. Ce produit de digestion sans autre purification a été ligué dans le vecteur d'expression pTG927 après coupure NdeI/PvuII. Parmi les transformants de E. coli TGE900, la construction correcte pTG726 a été identifiée par la présence d'un site unique BamHI dérivant de la séquence du gène HV1 et ensuite par une analyse de séquence d'ADN directe.

Expression de l'activité biologique de l'hirudine HV1 avec pTG726

Le vecteur d'expression pTG726 comporte un promoteur thermo-inductible, le promoteur $P_L$, promoteur majeur gauche du bactériophage λ. Comme ce promoteur est bloqué par un répresseur thermosensible codé par l'hôte, le gène de l'hirudine HV1 n'est pas transcrit pendant la croissance à 30° C. Toutefois, lorsque la température est élevée au-delà de 37° C, la transcription de ce gène est induite.

La figure 24 montre les courbes de croissance et l'induction de l'activité antithrombinique dans une culture de E. coli pTG726 mis en croissance à 30° C jusqu'à une densité optique de 0,3 à 600 nm, puis avec une induction à 37° C.

L'activité hirudine est mesurée par la capacité des extraits soniqués des cellules bactériennes d'inhiber l'activité de la thrombine bovine dans sa capacité à cliver des substrats chromogènes.

Il est clair que des quantités significatives d'hirudine sont induites dans les cultures de pTG726. Environ 3 à 4 000 unités antithrombine/DO/litre de culture, mais cette activité décline rapidement dans le temps. Cet effet est bien reproductible avec un pic d'activité 3 heures après l'induction, suivie d'une étape de déclin.

La nature de cette courbe d'induction est très caractéristique et reproductible et diffère significativement de celles qui ont été observées précédemment avec le vecteur d'expression pTG720 qui, par induction, exprime l'hirudine variant HV2. Ce variant est induit beaucoup plus lentement (figure 25A) avec un temps de latence d'environ 2 heures, l'activité augmente pour atteindre pratiquement le même niveau que celle de pTG726 (figure 25B) mais alors demeure constante sans aucune indication de déclin. Comme les deux variants d'hirudine ont été produits en utilisant exactement le même vecteur d'expression dans exactement la même cellule hôte E. coli TGE900, cette différence dans l'induction et la stabilité doit être certainement reliée à des différences dans les structures primaires entre HV1 et HV2. Ceci peut, en outre, refléter une différence dans les résistances à la digestion protéolitique entre les deux variants et peut être une indication d'activité biologique différente ou d'un rôle biologique différent pour les deux variants. De telles différences dans la stabilité et autres propriétés biologiques peuvent éventuellement être mises à profit dans l'utilisation de ces hirudines.

La différence dans l'expression de HV1 et HV2 dans E. coli peut se remarquer également par analyse "pulse-labelling" de cellules E. coli TGE900 transformées par deux vecteurs d'expression différents. Comme les deux variants hirudine sont très riches en cystéine (environ 10 % de la molécule), et comme cet amino-acide est assez rare dans les protéines de E. coli, la cystéine $35_S$ est très utile comme marqueur radioactif de l'expression de l'hirudine. Les cellules de E. coli transformées par pTG726 sont mises en croissance à 30° C jusqu'à une densité optique de 0,3 déterminée dans LB + ampicilline (100 µg/ml) puis induites pour exprimer le variant HV1 par augmentation de la température à 37° C.

A intervalle régulier d'une heure, on prélève un aliquote de 200 µl de la culture et on ajoute 70 µCi de cystéine $35_S$ (activité spécifique 1000 Ci/mmole) pendant une période de marquage de 2 minutes. Puis, un large excès, environ 2 ml, de tampon phosphate salin froid est ajouté, les cellules sont collectées par centrifugation et les protéines marquées des cellules entières sont analysées en faisant bouillir le culot pendant 5 minutes dans 40 µl d'un tampon de charge d'un gel SDS(50 mM Tris HCl, pH 6,8, 1,3 % SDS, 5 % glycérol, 2,5 % β-mercapto-éthanol, 0,004 % Bromophénol Blue) et charge de 5 µl sur un gel 15 % SDS-polyacrylamide (protocole de Laemli-réf. 36). Après électrophorèse, le gel est soumis à une fluorographie suivie par une autoradiographie.

Les résultats montrent une induction d'une série de bandes dans la région des 6 000 à 12 000 daltons, correspondant à l'hirudine. Ces bandes ne sont que faiblement marquées avec les extraits de E. coli/pTG726 (variant HV1) alors qu'elles sont très fortement marquées avec les extraits de E. coli/pTG720. Cette différence très nette dans la physionomie du marquage apparaît en dépit du fait que les deux cultures

présentent à peu près le même niveau d'activité antithrombinique.

Autres propriétés du recombinant HV1 de l'hirudine

L'une des caractéristiques de l'hirudine naturelle et également du variant HV2 préparé par E. coli est sa résistance au traitement par la chaleur dans des conditions de pH assez bas. Ceci est également valable pour le variant HV1 préparé à partir de E. coli. Une culture induite pendant 3 heures de cellules de E. coli transformées par pTG726 est collectée par centrifugation, resuspendue dans 1/5 du volume de culture de TGE (50 mM Tris HCl pH 8,0, 50 mM glucose, 10 mM EDTA) et les cellules sont brisées par sonication. Les débris cellulaires sont éliminés par centrifugation et une partie du surnageant est utilisée directement pour la détermination de l'activité antithrombinique.

Une autre partie est ajustée à pH 2,8 avec de l'acide HCl dilué puis chauffée à 70°C pendant 15 minutes. L'ensemble est alors refroidi sur de la glace pendant 30 minutes et les protéines insolubles dénaturées sont éliminées par centrifugation. Le surnageant est neutralisé par addition de NaOH dilué puis l'activité antithrombinique est mesurée. Après avoir pris en compte les petites modifications de volume dues à l'acidification et la neutralisation, on peut calculer que 100 % de l'activité originale survit à ce traitement acide/chaleur. C'est pourquoi le variant HV1 est identique à l'hirudine naturelle et au variant HV2.

L'activité hirudine HV2 peut également être complètement prélevée d'un extrait bactérien en utilisant de la thrombine liée de façon covalente à des billes de Sepharose. Un extrait de E. coli/pTG726 traité par acide et chaleur puis neutralisé et contenant 7,7 unités d'activité antithrombinique dans 200 µl est incubé avec 50 µl d'une suspension à 50 % de thrombine Sepharose pendant 15 minutes à 37°C. Les billes de thrombine-sepharose sont prélevées par centrifugation et le surnageant est testé pour son activité antithrombinique. Plus de 95 % de l'activité antithrombinique d'origine sont éliminés par traitement sepharose-thrombine. En conséquence le variant HV1 produit par les cellules de E. coli est capable de se lier à la thrombine fixée sur des billes de sepharose.

Les souches suivantes ont été déposées le 26 mars 1985 à la Collection Nationale de Cultures de Microorganismes (CNCM) - 28 rue du Docteur-Roux - 75724 PARIS CEDEX 15 :

E. Coli TGE900 transformée par pTG718
n° I-427

E. Coli TGE900 transformée par pTG720
n° I-428

E. Coli TGE900 transformée par pTG726
n° I-429

REFERENCES

1. Markwardt, F. (1955) Naturwissenschaften 42, 587.
2. Markwardt, F. (1957) Hoppe-Seylers Z. Physiol. Chem. 308, 147-156.
3. Markwardt, F. and Walsmann, P. (1967) Hoppe-Seylers Z. Physiol. Chem. 348, 1381-1386.
4. de la Llosa, Tertrin, C. and Jutisz, M. (1963) Bull. Soc. Chim. Biol. 45, 63-74.
5. Markwardt, F. (1970) in Methods in Enzymology, eds. Perlman, G.E. and Lorand, L., Academic Press., vol. 19, pp. 924-932.
6. Bagdy, D., Barabas, E. and Graf, L. (1973) Thrombosis Research 2, 229-238.
7. Graf, L., Patthy, A., Barabas, E.B., and Bagdy, D. (1973) Biochim. Biophys. Acta 310, 416-417.
8. Petersen, T.E., Roberts, H.R., Sottrup-Jensen, L., Magnusson. S. and Badgy, D. (1976) Protides Biol. Fluids, Proc. Colloq. vol. 23, pp. 145-149.
9. Dodt, J., Müller, H.-P., Seemüller, U., and CHang, J.-Y. (1984) Febs Lett. 165, 180-183.
10. Krajewski, T., and Blombäck, B. (1968) Acta. Chem. Scand. 22, 1339-1346.
11. Chang, J.-Y. (1983) Febs Lett. 164, 307-313.
12. Baskova, I.P., Cherkesova, D.U., Mosolov, V.V., Malova, E.L., and Belyanova, L.A. (1980) Biokhimiya 45, 463-467.
13. Baskova, I.P., Cherkesova, D.U., and Mosolov, V.V. (1983) Thrombosis Research 30, 459-467.
14. Markwardt, F., Hauptmann, J., Nowak, G., Klessen, Ch., and Walsmann, P. (1982) Thromb. Hemostasis (Stuttgart) 47, 226-229.
15. Walsmann, P. and Markwardt, F. (1981) Die Pharmazie 10, 653-660.
16. Kloss, Th., and Mittmann, U. (1982) Longenbecks Arch. Chirurg. 358, 548.
17. Ishikawa, A., Hafter, R., Seemüller, U., Gokel, J.M., and Graeff, M. (1980) Thrombosis Research 19, 351-358.

18. Nowak, G., and Markwardt, F. (1980) Expt. Path. 18, 438-443.
19. Sutor, A.H., Knop, S., and Adler, D. (1981) in Kontrolle Antithrombotica, 23rd Symp. Blutgerinnung, Hamburg, pp. 117-123.
20. Bagdy, D., Barabas, E., Graf, L., Petersen, T.E. and Magnusson, S. (1976) in Methods in Enzymology part B, vol. 45, pp. 669-678.
21. Walsmann, P. (1981) Pharmazie 36, 860-861.
22. Aviv, H., and Leder, P. (1972) Proc. Natl. Acad. Sci. USA 69, 1408-1412.
23. Jaye, M., De La Salle, H., Schamber, F., Balland, A., Kohli, V., Findeli, A., Tolstoshev, P. and Lecocq, J.P. (1983) Nucleic Acids Res. 11, 2325-2335.
24. Szostak, J.W., Stiles, J.I., Tye, B.-K., Chiu, P., Sherman, F. and Wu, R. (1979) in Methods in Enzymology, vol. 68, pp. 419-428.
25. Kohli, V., Balland, A., Wintzerith, M., Sauerwald, R., Staub, A. and Lecocq, J.P. (1982) Nucl. Acids Res. 10, 7439-7448.
26. Sanger, F., Nicklen, S. and Coulson, A.R. (1977) Proc. Natl. Acad. Sci. USA 74, 5463-5467.
27. Thomas, P. (1980) Proc. Natl. Acad. Sci. USA 77, 5201-5205.
28. Kalckar, H. (1947) J. Biol. Chem. 167, 461.
29. Caen, J., Larrieu, M.J. and Samama, M. (1975) in "L'Hémostase, Méthode d'exploration et diagnostic pratique" 2ème édition, Ed. L'Expansion Scientifique, Paris.
30. Vieira J., Messing J., Gene 19 , 259-268.
31. Kohli V., Balland A., Wintzerith M., Sauerwald R., Staub A. et Lecocq J-P, (1982) Nucl. Acids Res. 10, 7439-7448.
32. Grantham R., Gauthier C., Gouy M., Jacobzon E.M. et Mercier R. (1981) Nucl. Acids Res. 9, 143-174.
33. Ikemura T. (1981) J. Molec. Biol. 151, 389-409.
34. Sanger F., Coulson A.R., Barrel B.G., Smith A.J.H. et Roe B.A., J. Mol. Biol. (1980) 161-178.
35. Birnboim H.C. et Doly S., Nucl. Acids Res. (1979) 7, 1513.
36. Laemli U., Nature (1970) 227, 680-685.

**Revendications**
**Revendications pour les Etats contractants suivants : BE, CH, DE, FR, GB, IT, LI, LU, NL, SE**

**1.** Hirudine comportant tout ou partie du peptide :

Ile Thr Tyr Thr Asp Cys Thr Glu Ser Gly Gln Asn Leu Cys Leu Cys Glu Gly
Ser Asn Val Cys Gly Lys Gly Asn Lys Cys Ile Leu Gly Ser Asn Gly Lys Gly
Asn Gln Cys Val Thr Gly Glu Gly Thr Pro Asn Pro Glu Ser His Asn Asn Gly
Asp Phe Glu Glu Ile Pro Glu Glu Tyr Leu Gln

ainsi que son variant :
1. Ile→Val
2. Thr→Val.

**2.** Hirudine selon la revendication 1, caractérisée en ce qu'elle ne comporte pas de glycosylation ou de sulfatation.

**3.** Vecteur de clonage et d'expression dans une cellule hôte de l'hirudine ou d'un analogue de l'hirudine, caractérisé en ce qu'il comporte le gène codant pour l'hirudine ou un analogue de l'hirudine, ce gène codant débutant par une séquence codant, après la séquence de départ, pour Ile et Thr.

**4.** Vecteur selon la revendication 3, caractérisé en ce qu'il comporte la séquence suivante au départ du gène de l'hirudine :

ATG ATT ACG
TAC TAA TGC

5. Vecteur selon la revendication 4, caractérisé en ce qu'il comporte la séquence suivante au départ du gène de l'hirudine :

ATG ATT ACG TAT ACA GAC TGC ACA
TAC TAA TGC ATA TGT CTG ACG TGT

6. Vecteur pTG720 déposé dans la souche E. coli TGE900 à la CNCM sous le nº 1-428.

7. Cellule transformée par un vecteur selon l'une des revendications 3 à 6.

8. Cellule selon la revendication 7, caractérisée en ce qu'il s'agit d'une bactérie.

9. Cellule selon la revendication 8, caractérisée en ce qu'il s'agit d'une souche de E. coli.

10. Procédé de préparation d'hirudine ou d'un analogue d'hirudine, caractérisé en ce qu'on cultive une cellule transformée selon l'une des revendications 7 à 9 et que l'on récupère l'hirudine ou l'analogue de l'hirudine formés.

11. Procédé selon la revendication 10, caractérisé en ce que l'hirudine est récupérée à partir du produit de culture par chauffage à pH acide suivi d'une décantation, l'hirudine ou son analogue étant récupéré dans le surnageant.

12. Procédé selon l'une des revendications 10 et 11, caractérisé en ce que l'hirudine est récupérée par fixation sur une résine portant de la thrombine, puis élution de la réinse après fixation avec un compétiteur de l'hirudine.

13. Hirudine ou analogue de l'hirudine susceptibles d'être obtenus par la mise en oeuvre du procédé selon l'une des revendications 10 à 12.

14. Utilisation de l'hirudine selon l'une des revendications 1 et 2 et 13, marquée pour la fabrication d'un agent de diagnostic de la formation des caillots chez l'homme ou l'animal.

15. Circuit extracorporel sanguin caractérisé en ce qu'au moins une partie du circuit en contact avec le sang est revêtue d'hirudine selon l'une des revendications 1 et 2 et 13.

16. Procédé de séparation d'un facteur de coagulation à partir de sang ou d'une fraction sanguine, caractérisé en ce que ladite séparation est effectuée en présence d'hirudine selon l'une des revendications 1 et 2 et 13.

**Revendications pour l'Etat contractant suivant :AT**

1. Procédé de préparation d'hirudine ou d'un analogue d'hirudine, caractérisé en ce que l'on cultive une cellule transformée par un vecteur de clonage et d'expression comportant un gène codant pour l'hirudine ou un analogue de l'hirudine, ce gène codant débutant par une séquence codant, après la séquence de départ, pour Ile et Thr, et qu'on récupère l'hirudine ou l'analogue de l'hirudine formée.

2. Procédé selon la revendication 1, **caractérisé** en ce que la cellule transformée est une bactérie.

3. Procédé selon la revendication 2, **caractérisé** en ce qu'il s'agit d'une souche de E. Coli.

4. Procédé selon l'une des revendications 1 à 3, **caractérisé** en ce que le vecteur de clonage comporte la séquence suivante au départ du gène de l'hirudine :

ATG ATT ACG
TAC TAA TGC

5. Procédé selon la revendication 4, **caractérisé** en ce qu'elle comporte la séquence suivante au départ du gène de l'hirudine :

ATG ATT ACG TAT ACA GAC TGC ACA
TAC TAA TGC ATA TGT CTG ACG TGT

6. Procédé selon l'une des revendications 1 à 5, **caractérisé** en ce que l'hirudine ou son analogue est récupéré à partir du produit de culture par chauffage à pH acide suivi d'une décantation, l'hirudine ou son analogue étant récupéré dans le surnageant.

7. Procédé selon l'une des revendications 1 à 6, **caractérisé** en ce que l'hirudine est récupérée par fixation sur une résine portant de la thrombine, puis élution de la résine après fixation avec un compétiteur de l'hirudine.

8. Procédé selon l'une des revendications 1 à 7, **caractérisé** en ce que le vecteur de clonage est le vecteur pTG720 déposé dans la souche E. Coli TGE900 à la CNCM sous le n° I-428.

**Claims**
**Claims for the following Contracting States : BE, CH, DE, FR, GB, IT, LI, LU, NL, SE**

1. Hirudin containing all or part of the following peptide:

Ile Thr Tyr Thr Asp Cys Thr Glu Ser Gly Gln Asn Leu Cys Leu Cys Glu Gly
Ser Asn Val Cys Gly Lys Gly Asn Lys Cys Ile Leu Gly Ser Asn Gly Lys Gly
Asn Gln Cys Val Thr Gly Glu Gly Thr Pro Asn Pro Glu Ser His Asn Asn Gly
Asp Phe Glu Glu Ile Pro Glu Glu Tyr Leu Gln

as well as its variant:
1. Ile→Val
2. Thr→Val

2. Hirudin as claimed in claim 1, characterized in that it does not comprise glycosylation or sulfatation.

3. Vector for cloning and expression of hirudin or a hirudin analog in a host cell, characterized in that it contains the gene coding for hirudin or a hirudin analog said coding gene begining with a sequence coding, after the starting sequence, for Ile and Thr.

4. Vector as claimed in claim 3, characterized in that it contains the following sequence at the start of the hirudin gene:

ATG ATT ACG
TAC TAA TGC

5. Vector as claimed in claim 4, characterized in that it contains the following sequence at the start of the

hirudin gene:

ATG ATT ACG TAT ACA GAC TGC ACA
TAC TAA TGC ATA TGT CTG ACG TGT

**6.** Vector pTG 720 deposited in the strain E. coli TGE 900 at the CNCM under the nº I-428.

**7.** Cell transformed by a vector as claimed in one of claims 3 to 6.

**8.** Cell as claimed in claim 7, characterized in that it is a bacterium.

**9.** Cell as claimed in claim 8, characterized in that it is a strain of E. coli.

**10.** Process for the preparation of hirudin or a hirudin analog, characterized in that a transformed cell as claimed in one of claims 7 to 9 is cultured and wherein the hirudin or hirudin analog formed is recovered.

**11.** Process as claimed in claim 10, characterized in that the hirudin is recovered from the culture product by heating at acid pH followed by decantation, the hirudin or its analog being recovered by decantation, the hirudin or its analog being recovered in the supernatant.

**12.** Process as claimed in either of claims 10 and 11, characterized in that the hirudin is recovered by binding it to a resin bearing thrombin, followed by elution of the resin after binding with a hirudin competitor.

**13.** Hirudin or hirudin analog obtained by carrying out the process as claimed in one of claims 10 to 12.

**14.** Use of hirudin as claimed in one of claims 1 and 2 and 13 which is labeled for manufacturing an agent for the diagnosis of clot formation in man or animals.

**15.** Extracorporeal blood circuit, wherein at least part of the circuit in contact with the blood is coated with hirudin as claimed in one of claims 1 and 2 and 13.

**16.** Process for the separation of a clotting factor from blood or from a blood fraction, characterized in that said separation is performed, in the presence of hiruding as claimed in one of claims 1 and 2 and 13.

**Claims for the following Contracting State : AT**

**1.** Process for the preparation of hirudin or a hirudin analog, characterized in that a cell transformed by a vector for cloning and expression containing a gene coding for hirudin or a hirudin analog, said coding gene begining with a sequence coding, after the starting sequence, for Ile and Thr, is cultured, and the hirudin or hirudin analog is recovered.

**2.** Process as claimed in claim 1, characterized in that the tranformed cell is a bacterium.

**3.** Process as claimed in claim 2, characterized in that it is a strain of E. coli.

**4.** Process as claimed in one of claims 1 to 3, characterized in that the vector for cloning contains the following sequence at the start of the hirudin gene:

ATG ATT ACG
TAC TAA TGC

**5.** Process as claimed in claim 4, characterized in that it contains the following sequence at the start of the hirudin gene:

ATG ATT ACG TAT ACA GAC TGC ACA
TAC TAA TGC ATA TGT CTG ACG TGT

**6.** Process as claimed in one of claims 1 to 5, characterized in that hirudin or its analog is recovered from the culture product by heating at acid pH followed by decantation, the hirudin or its analog being recovered in the supernatant.

**7.** Process as claimed in one of claims 1 to 6, characterized in that the hirudin is recovered by binding it to a resin bearing thrombin, followed by elution of the resin after binding with a hirudin competitor.

**8.** Process as claimed in one of clams 1 to 7, characterized in that the vector for cloning is the vector PTG 720 deposited in the strain E. coli TGE 900 at the CNCM under the n° I.428.

**Patentansprüche**
**Patentansprüche für folgende Vertragsstaaten : BE, CH, DE, FR, GB, IT, LI, LU, NL, SE**

**1.** Hirudin, umfassend das gesamte oder einen Teil des Peptids:

Ile Thr Tyr Thr Asp Cys Thr Glu Ser Gly Gln Asn Leu Cys Leu Cys Glu
Gly Ser Asn Val Cys Gly Lys Gly Asn Lys Cys Ile Leu Gly Ser Asn Gly
Lys Gly Asn Gln Cys Val Thr Gly Glu Gly Thr Pro Asn Pro Glu Ser His
Asn Asn Gly Asp Phe Glu Glu Ile Pro Glu Glu Tyr Leu Gln

sowie seine Variante:
1. Ile→Val
2. Thr→Val.

**2.** Hirudin nach Anspruch 1, dadurch gekennzeichnet, daß es keine Glykolisierung oder Sulfatierung aufweist.

**3.** Glonierungs- und Expressions-Vektor in einer Wirtszelle, für Hirudin oder ein Analoges von Hirudin, dadurch gekennzeichnet, daß er das für Hirudin oder ein Analoges von Hirudin kodierende Gen umfaßt, wobei dieses kodierende Gen mit einer Sequenz beginnt, die nach der Ausgangssequenz für Ile und Thr kodiert.

**4.** Vektor nach Anspruch 3, dadurch gekennzeichnet, daß er folgende Sequenz am Anfang des Gens für Hirudin umfaßt:

ATG ATT ACG
TAC TAA TGC.

**5.** Vektor nach Anspruch 4, dadurch gekennzeichnet, daß er folgende Sequenz am Anfang des Gens für Hirudin umfaßt:

ATG ATT ACG TAT ACA GAC TGC ACA

TAC TAA TGC ATA TGT CTG ACG TGT.

**6.** Vektor pTG720, hinterlegt im Stamm E. coli TGE900 bei CNCM unter der Nummer I-428.

**7.** Zelle, transformiert durch einen Vektor nach einem der Ansprüche 3 bis 6.

**8.** Zelle nach Anspruch 7, dadurch gekennzeichnet, daß es sich um eine Bakterie handelt.

**9.** Zelle nach Anspruch 8, dadurch gekennzeichnet, daß es sich um einen Stamm von E. coli handelt.

**10.** Verfahren zur Herstellung von Hirudin oder einem Analogen von Hirudin, dadurch gekennzeichnet, daß man eine transformierte Zelle nach einem der Ansprüche 7 bis 9 kultiviert und daß man das gebildete Hirudin oder das Analoge von Hirudin gewinnt.

**11.** Verfahren nach Anspruch 10, dadurch gekennzeichnet, daß das Hirudin aus dem Kulturprodukt gewonnen wird durch Erwärmen bei saurem pH, gefolgt von einem Dekantieren, wobei das Hirudin oder sein Analoges aus der überstehenden Flüssigkeit gewonnen werden.

**12.** Verfahren nach einem der Ansprüche 10 und 11, dadurch gekennzeichnet, daß das Hirudin durch Fixieren auf einem Thrombin tragenden Harz und anschließendes Eluieren des Harzes nach dem Fixieren mit einem mit Hirudin konkurrierenden Material gewonnen wird.

**13.** Hirudin oder Analoges von Hirudin, erhältlich durch Durchführung des Verfahrens nach einem der Ansprüche 10 bis 12.

**14.** Verwendung von Hirudin nach einem der Ansprüche 1 und 2 und 13, markiert, zur Herstellung eines diagnostischen Mittels für die Bildung von Blutklümpchen bei Mensch und Tier.

**15.** Extracorporaler Blutkreislauf, dadurch gekennzeichnet, daß zumindest ein Teil des mit dem Blut in Kontakt befindlichen Kreislaufs mit Hirudin gemaß einem der Ansprüche 1 und 2 und 13 ausgekleidet ist.

**16.** Verfahren zur Abtrennung eines Koagulationsfaktors von Blut oder einer Blutfraktion, dadurch gekennzeichnet, daß die Abtrennung in Anwesenheit von Hirudin gemäß einem der Ansprüche 1 und 2 und 13 durchgeführt wird.

**Patentansprüche für folgenden Vertragsstaat : AT**

**1.** Verfahren zur Herstellung von Hirudin oder einem Analogen von Hirudin, dadurch gekennzeichnet, daß man eine durch einen Clonierungs- und Expressions-Vektor transformierte Zelle kultiviert, die ein Gen aufweist, das für Hirudin oder ein Analoges von Hirudin kodiert, wobei dieses kodierende Gen mit einer Sequenz beginnt, die nach der Ausgangssequenz für Ile und Thr kodiert, und daß man das gebildete Hirudin oder Analoge von Hirudin gewinnt.

**2.** Verfahren nach Anspruch 1, dadurch gekennzeichnet, daß die transformierte Zelle eine Bakterie ist.

**3.** Verfahren nach Anspruch 2, dadurch gekennzeichnet, daß es sich um einen Stamm von E. coli handelt.

**4.** Verfahren nach einem der Ansprüche 1 bis 3, dadurch gekennzeichnet, daß der Clonierungsvektor die folgende Sequenz am Beginn des Gens für Hirudin aufweist:

ATG ATT ACG

TAC TAA TGC.

5. Verfahren nach Anspruch 4, dadurch gekennzeichnet, daß es die folgende Sequenz am Beginn des Gens für Hirudin aufweist:

ATG ATT ACG TAT ACA GAC TGC ACA

TAC TAA TGC ATA TGT CTG ACG TGT.

6. Verfahren nach einem der Ansprüche 1 bis 5, dadurch gekennzeichnet, daß das Hirudin oder sein Analoges aus dem Kulturprodukt durch Erwärmen bei saurem pH, gefolgt von einem Dekantieren, gewonnen wird, wobei das Hirudin oder sein Analoges aus der überstehenden Flüssigkeit gewonnen werden.

7. Verfahren nach einem der Ansprüche 1 bis 6, dadurch gekennzeichnet, daß das Hirudin durch Fixieren an ein Harz, das Thrombin trägt, und anschließendes Eluieren des Harzes nach dem Fixieren mit einem mit Hirudin konkurrierenden Material gewonnen wird.

8. Verfahren nach einem der Ansprüche 1 bis 7, dadurch gekennzeichnet, daß der Clonierungsvektor der Vektor pTG720, hinterlegt im Stamm E. coli TGE900 bei CNCM unter der Nummer I-428, ist.

```
                                        10                         20
val-val-tyr-thr-asp-cys-thr-glu-ser-gly-gln-asn-leu-cys-leu-cys-glu-gly-ser-asn-
                                        30                         40
val-cys-gly-gln-gly-asn-lys-cys-ile-leu-gly-ser-asp-gly-glu-lys-asn-gln-cys-val-
                                        50                         60
thr-gly-glu-gly-thr-pro-lys-pro-gln-ser-his-asn-asp-gly-asp-phe-glu-glu-ile-pro-

glu-glu-tyr-leu-gln
        |
        SO3H
```

FIG_1

```
               49                                           34
(a) COOH --- glnprolysprothrglygluglythrvalcysglnasnlysglugly ---NH2

(b)       5' CTGAGGCTTAGGAGTACCCTGGCCGGTGACGCACTGGTTCT/CTCGCC 3'
             ● ●●●●●● ●● ●●●●● ● ●●● ●●●●● ●● ●●●● ● ● ● ●●
(c)       3' AAGTCCGAAGCCACATGGAAGCGGTCACTGTGTAACCAGGGGGGGGGG 5'

(d) COOH --- [glu]prolysprothrglygluglythrvalcysgln  |  polyG
```

FIG_2

1 GCA ATC TGC GTG TCT CAA GCA ATT ACT TAC 31 ACT GAT TGT ACA GAA TCG GGT CAA AAT TTG 61 TGC CTC TGC GAG GGA AGC AAT GTT TGC GGT
Ala Ile Cys Val Ser Gln Ala Ile Thr Tyr Thr Asp Cys Thr Glu Ser Gly Gln Asn Leu Cys Leu Cys Glu Gly Ser Asn Val Cys Gly

91 AAA GGC AAT AAG TGC ATA TTG GGT TCT AAT 121 GGA AAG GGC AAC CAA TGT GTC ACT GGC GAA 151 GGT ACA CCG AAC CCT GAA AGC CAT AAT AAC
Lys Gly Asn Lys Cys Ile Lou Gly Ser Asn Gly Lys Gly Asn Gln Cys Val Thr Gly Glu Gly Thr Pro Asn Pro Glu Ser His Asn Asn

181 GGC GAT TTC GAA GAA ATT CCA GAA GAA TAT 211 TTA CAA TGA AAA ATG AAA GAA TAT CAA TCA 241 TAG AGA ATT TTG ATT TAA AAA CAT TTC CAT
Gly Asp Phe Glu Glu Ile Pro Gln Glu Tyr Leu Gln •••

271 AGCTAAGCTATTTACCAATAAATAAATTA 301 ATTTTTCCATTGAATCTCAATCATATTTA 331 CTCTCAATCATATTCAGCTATTTACCAATA 361 AATAAATTAATTTTTCCATGA

FIG_3

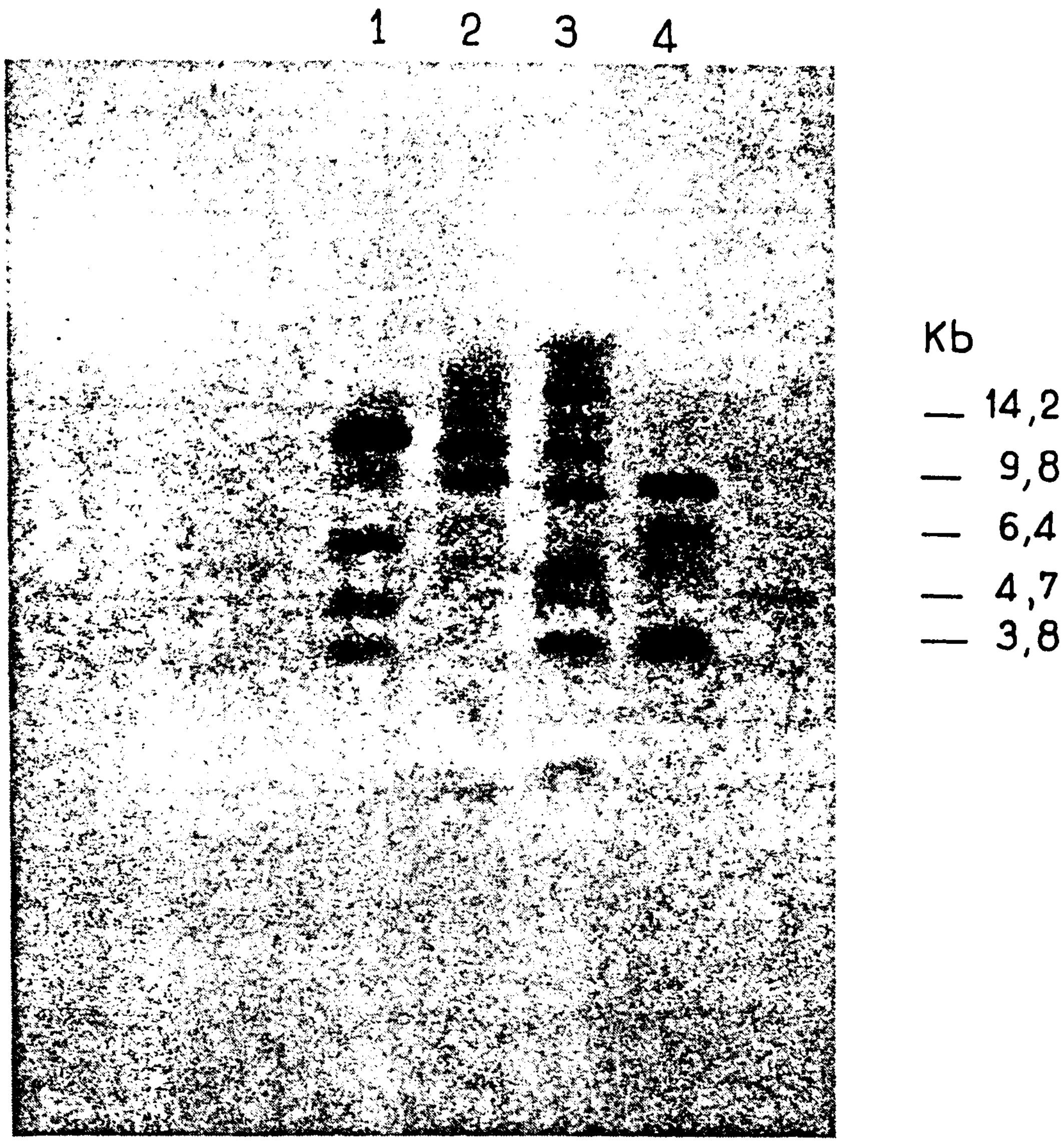


FIG_4

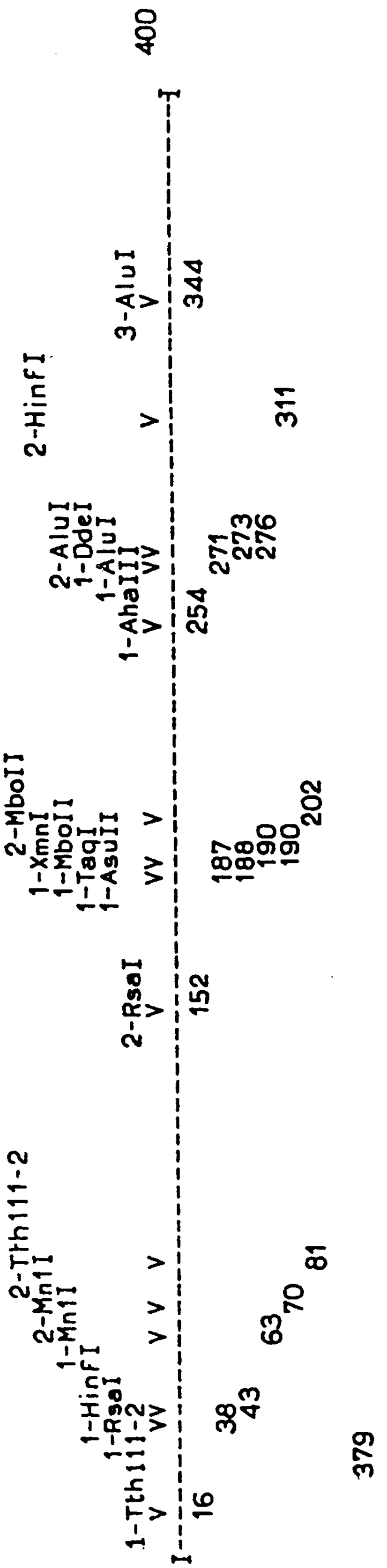
1
400
1-Tth111-2
1-RsaI
1-HinfI
1-MnlI
2-MnlI
2-Tth111-2
2-RsaI
1-XmnI
1-MboII
1-TaqI
1-AsuII
2-MboII
1-AhaIII
2-AluI
1-DdeI
1-AluI
2-HinfI
3-AluI
16
38
43
63
70
81
379
152
187
188
190
190
202
254
271
273
276
311
344

FIG.5

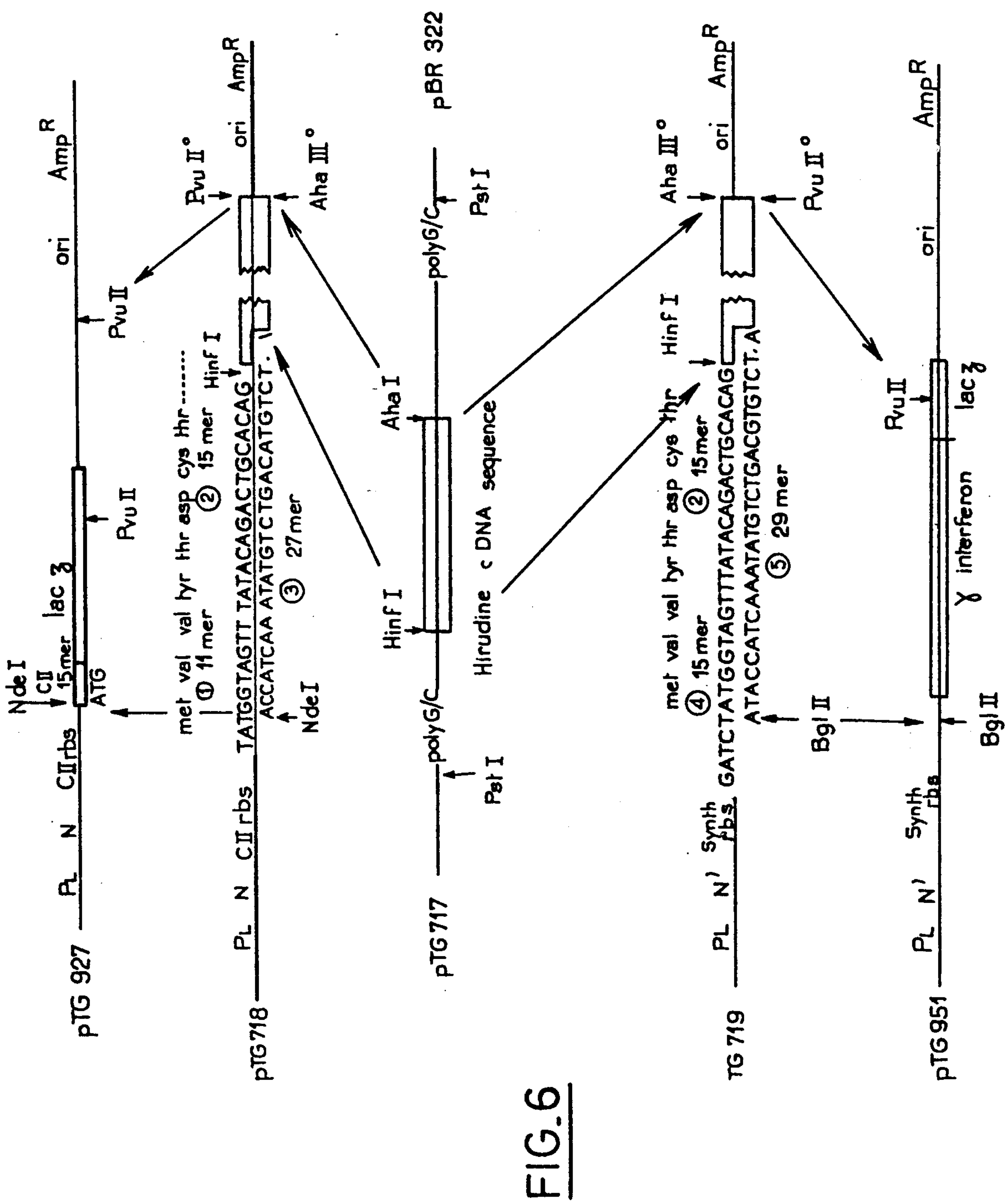
pTG 927
PL
N
CII rbs
Nde I
CII
15mer
ATG
lac Z
Pvu II
ori
AmpR
pTG718
met val val tyr thr asp cys thr -------
① 11 mer
② 15 mer
TATGGTAGTT TATACAGACTGCACAG
ACCATCAA ATATGTCTGACATGTCT.
③ 27 mer
Hinf I
Pvu II°
Aha III°
pTG717
polyG/C
Pst I
Hinf I
Aha I
Hirudine c DNA sequence
pBR 322
TG 719
N'
Synth rbs
met val val tyr thr asp cys thr
④ 15 mer
GATCTATGGTAGTTTATACAGACTGCACAG
ATACCATCAAATATGTCTGACGTGTCT.A
⑤ 29 mer
Bgl II
pTG951
γ interferon

FIG.6

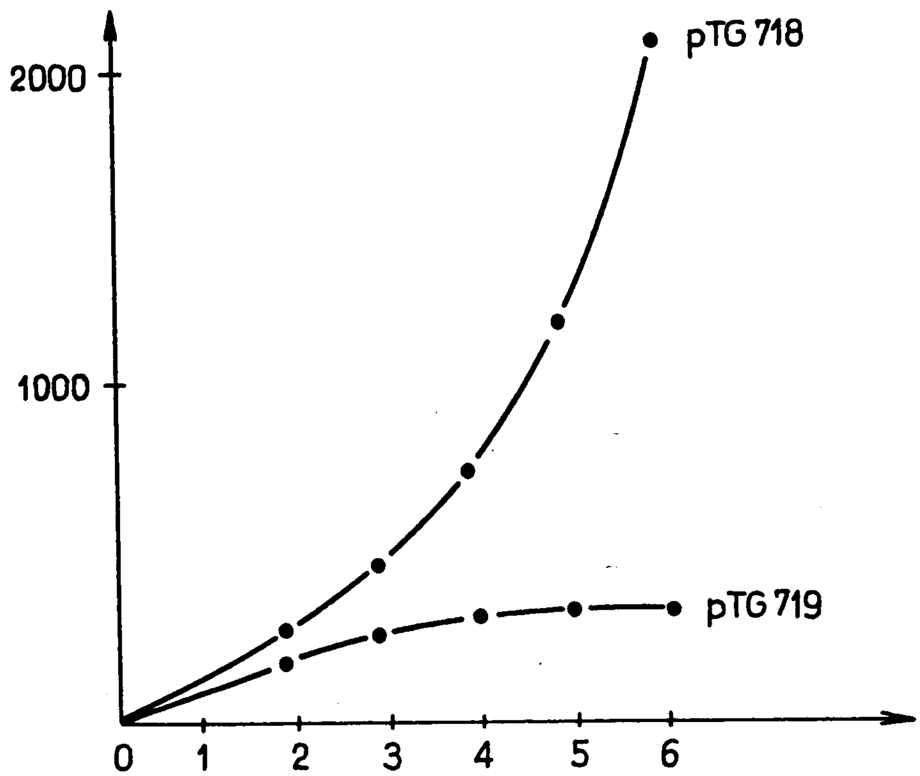


FIG_7

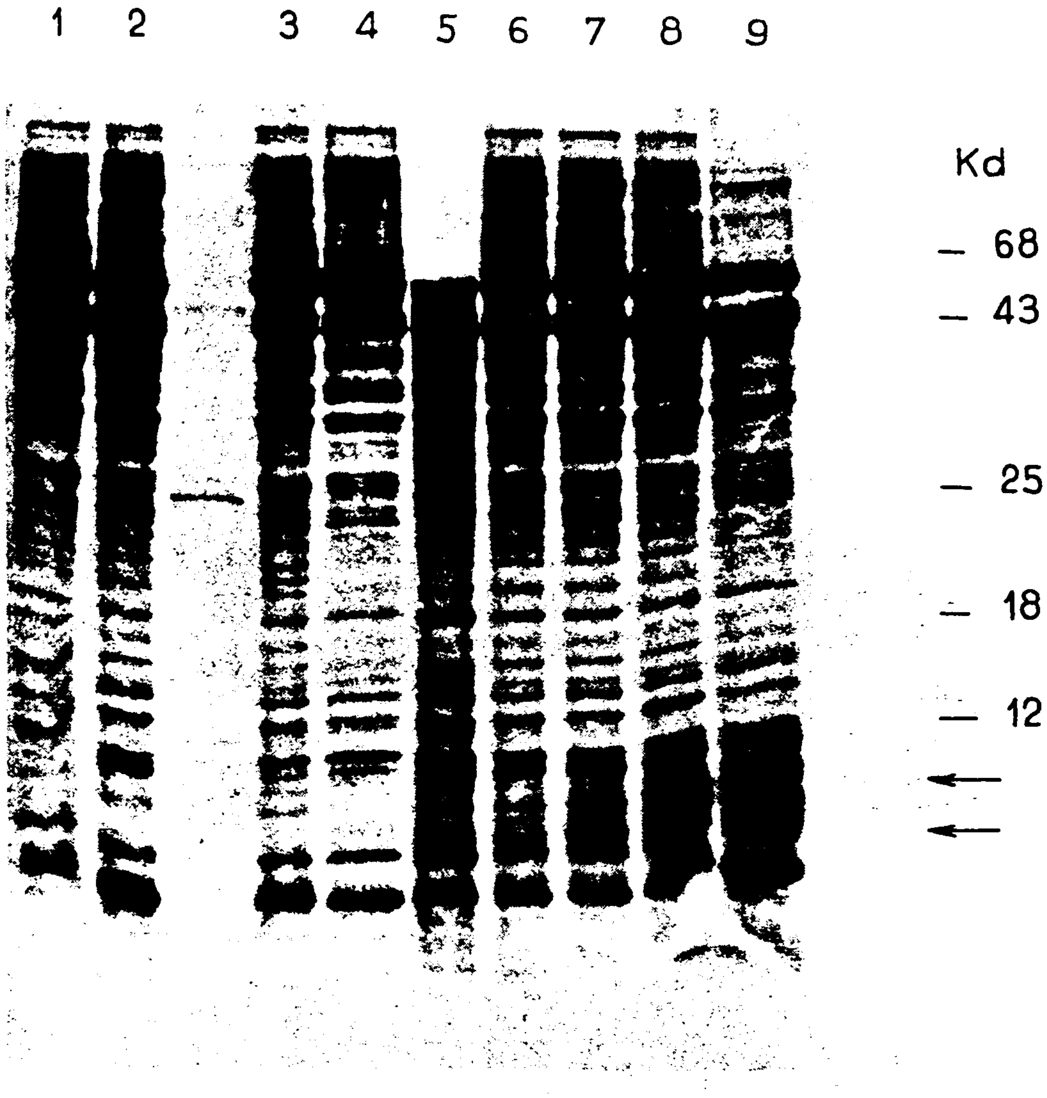


FIG.8

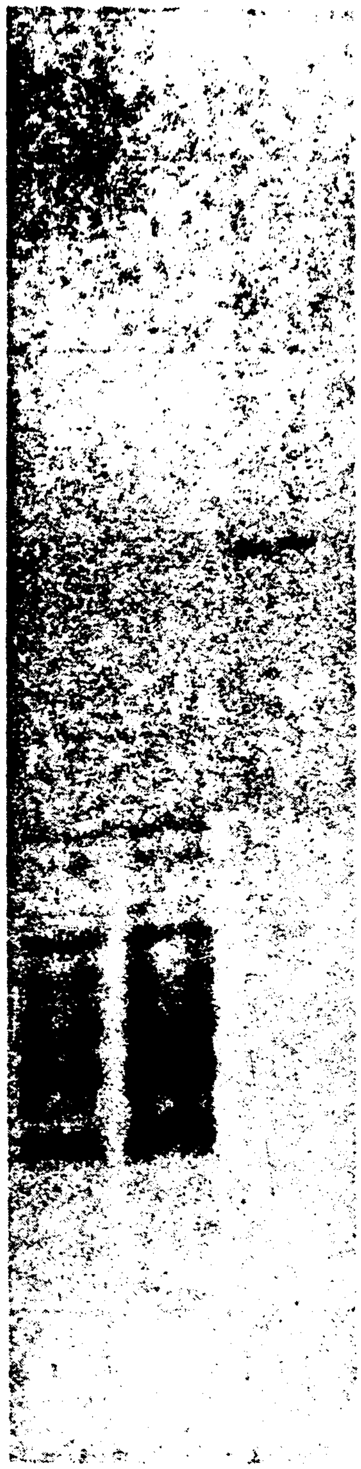

FIG_9

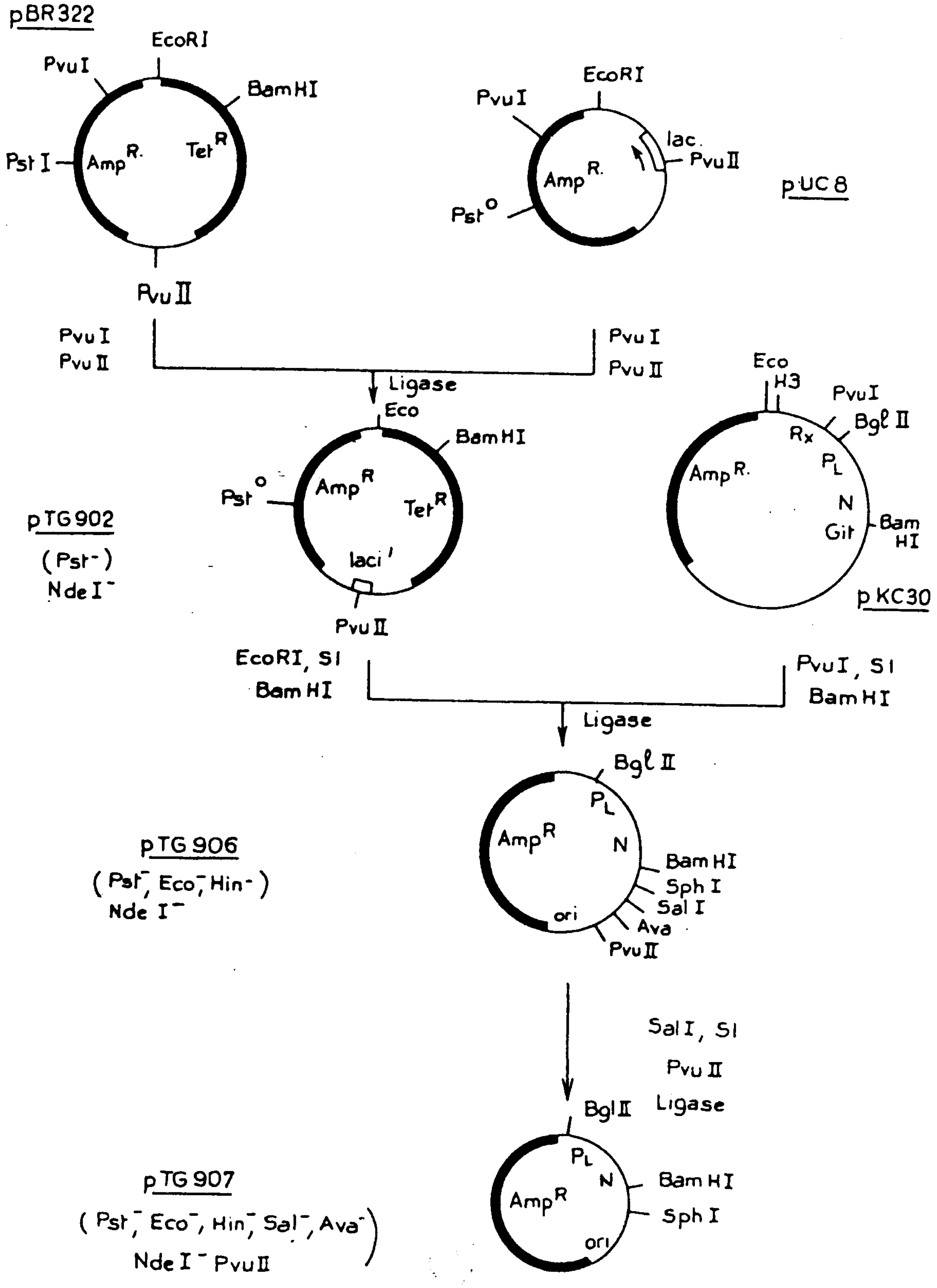
pBR322
EcoRI
PvuI
BamHI
PstI
AmpR
TetR
PvuII
EcoRI
PvuI
lac
PvuII
AmpR
Pst°
pUC8
PvuI
PvuII
PvuI
PvuII
Ligase
Eco
BamHI
Pst°
AmpR
TetR
laci
PvuII
pTG902
(Pst-)
NdeI-
Eco
H3
PvuI
BglII
Rx
PL
AmpR
N
Git
Bam HI
pKC30
EcoRI, SI
BamHI
PvuI, SI
BamHI
Ligase
BglII
PL
AmpR
N
BamHI
SphI
SalI
Ava
ori
PvuII
pTG906
(Pst-, Eco-, Hin-)
NdeI-
SalI, SI
PvuII
Ligase
BglII
PL
N
AmpR
BamHI
SphI
ori
pTG907
(Pst-, Eco-, Hin-, Sal-, Ava-)
NdeI- PvuII

FIG_10

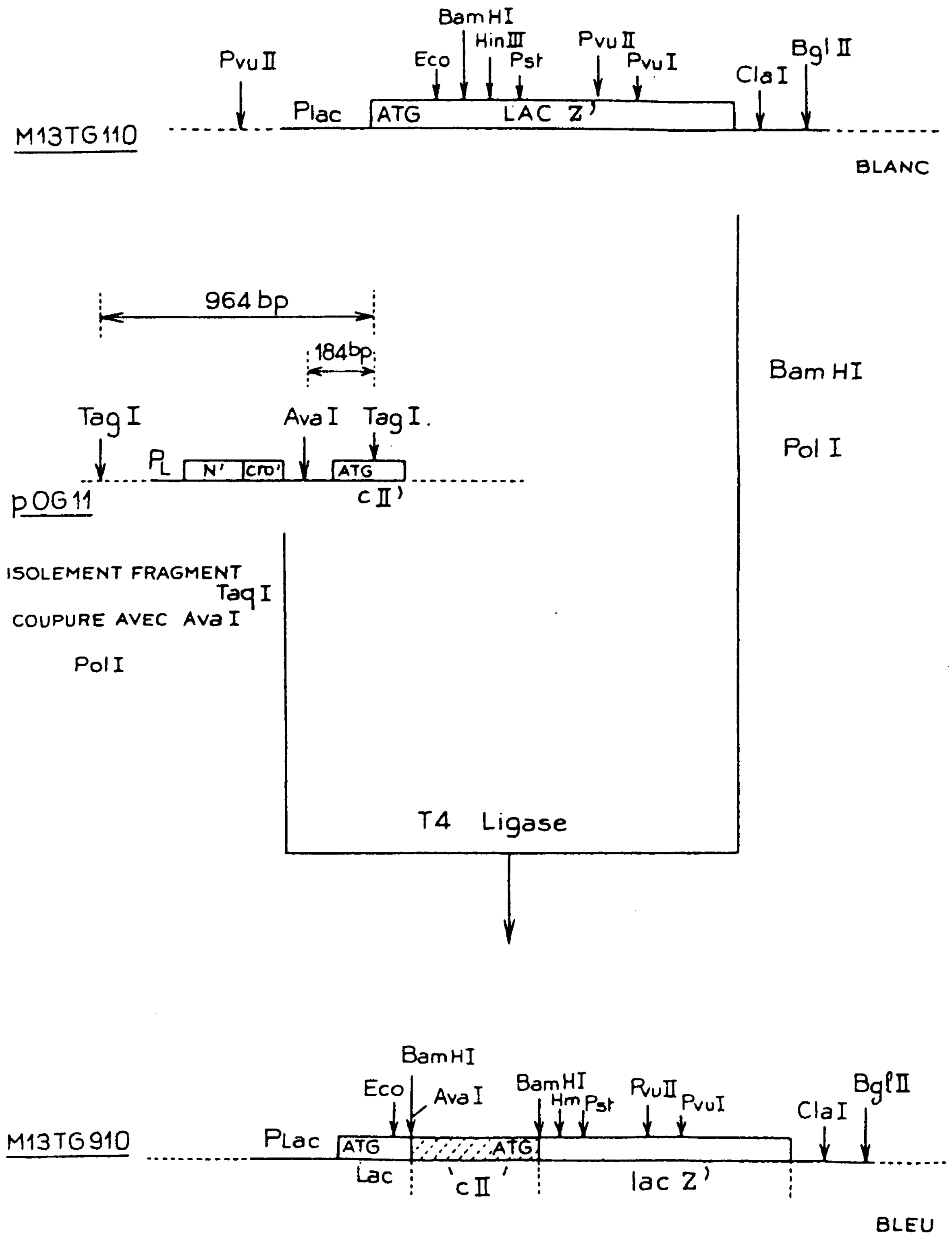
Bam HI
Hin III
Eco
Pst
Pvu II
Pvu I
Pvu II
Bgl II
Cla I
Plac
ATG
LAC Z'
M13TG110
BLANC
964 bp
184bp
Tag I
Ava I
Tag I.
PL
N'
cro'
ATG
c II'
pOG11
ISOLEMENT FRAGMENT
Taq I
COUPURE AVEC Ava I
Pol I
Bam HI
Pol I
T4 Ligase
Bam HI
Eco
Ava I
Bam HI
Hm
Pst
Pvu II
Pvu I
Bgl II
Cla I
M13TG910
Plac
ATG
ATG
Lac
'c II'
lac Z'
BLEU

FIG_11

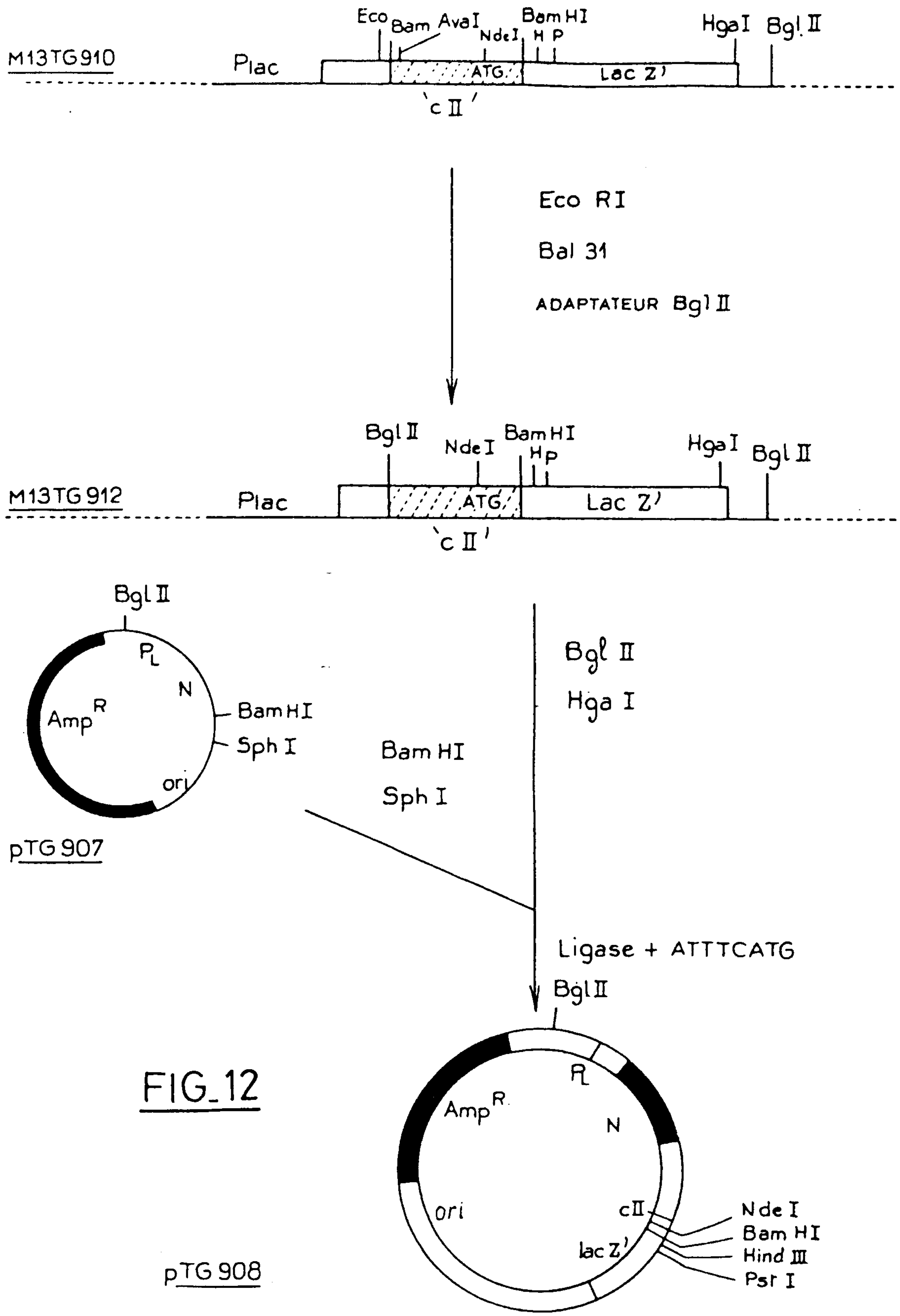
M13TG910
Plac
Eco
Bam
AvaI
NdeI
BamHI
H P
ATG
Lac Z'
HgaI
BglII
'cII'
Eco RI
Bal 31
ADAPTATEUR BglII
M13TG912
Plac
BglII
NdeI
BamHI
H P
ATG
Lac Z'
HgaI
BglII
'cII'
BglII
$P_L$
N
Amp$^R$
BamHI
Sph I
ori
pTG907
Bam HI
Sph I
Bgl II
Hga I
Ligase + ATTTCATG
BglII
$P_L$
Amp$^R$
N
ori
cII
lac Z'
Nde I
Bam HI
Hind III
Pst I
pTG908

FIG_12

CONSTRUCTION DE PTG909

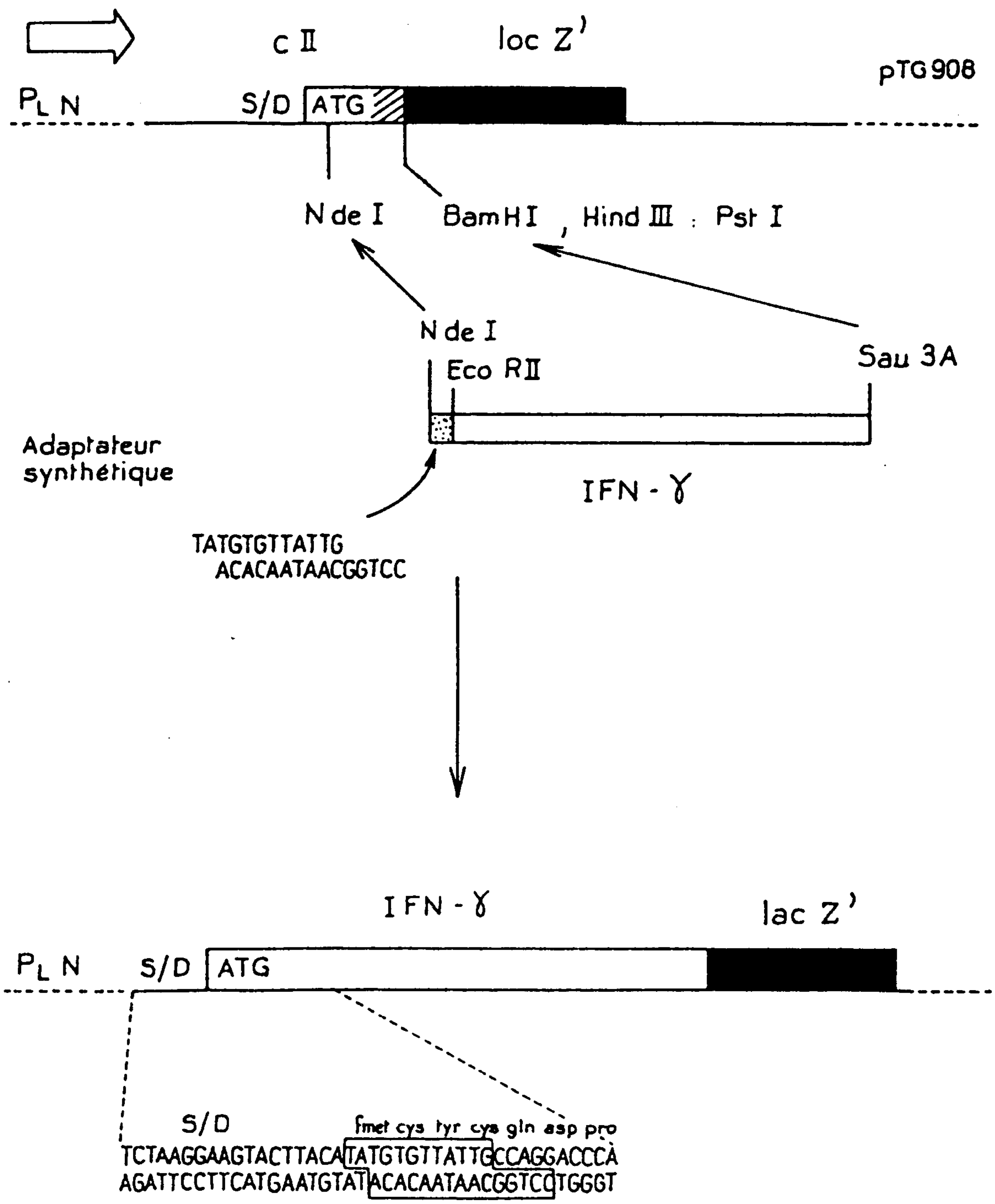


FIG_13

CONSTRUCTION DE PTG941

PTG909

IFN

*MetCysTyrCysGlnAspProTyr*

TAAGGAAGTACTTACATATGTGTTATTGCCAGGACCCATATG....

NDEI NDEI

NDEI

\+

TATGTGCTACTGTCAGGATCCC

ACACGATGACAGTCCTAGGGAT

IFN

PTG941

*MetCysTyrCysGlnAspProTyr*

TAAGGAAGTACTTACATATGTGCTACTGTCAGGATCCCTAT

NDEI BAMHI

FIG_14

CONSTRUCTION DE PTG951

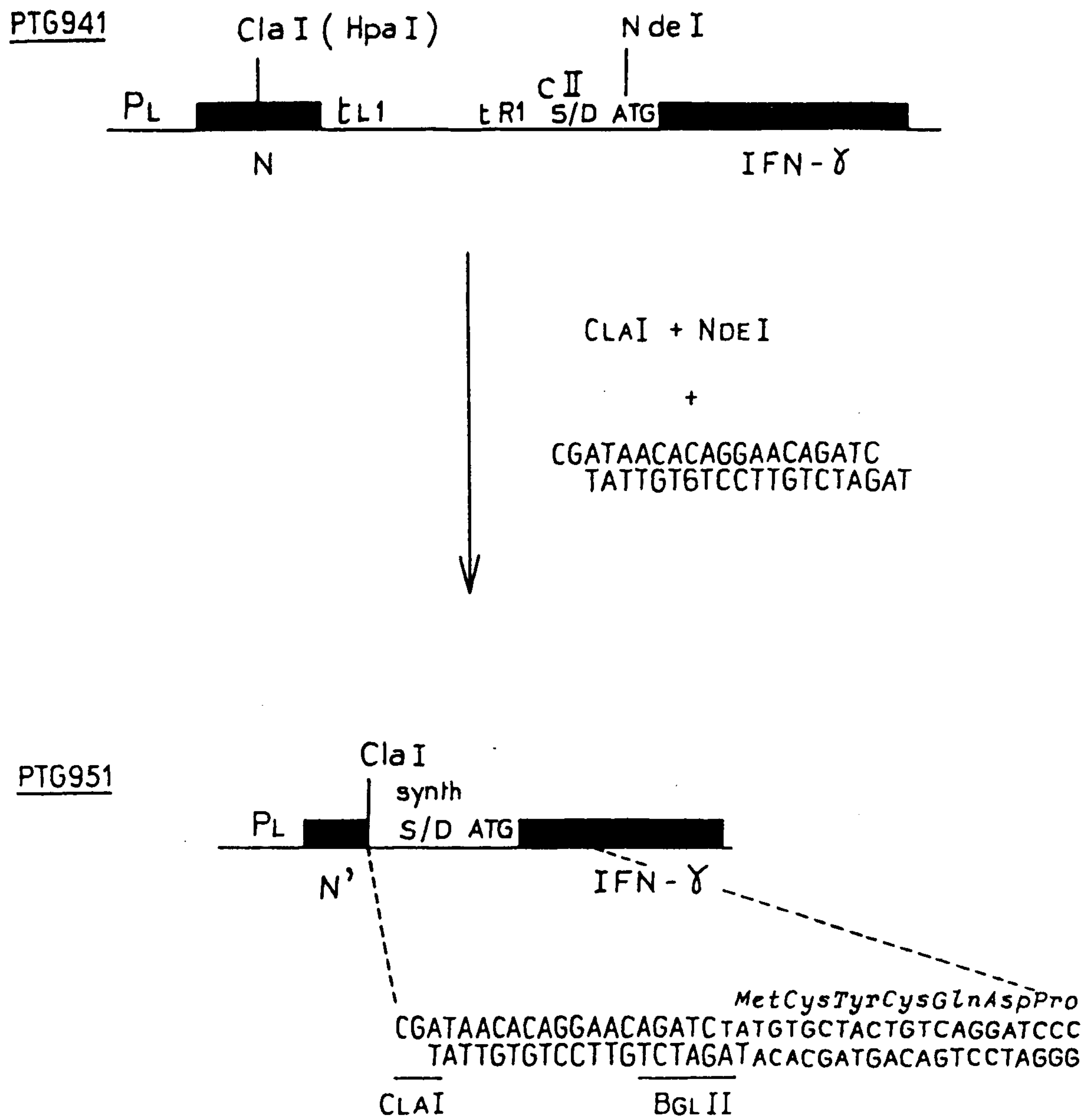


FIG_15

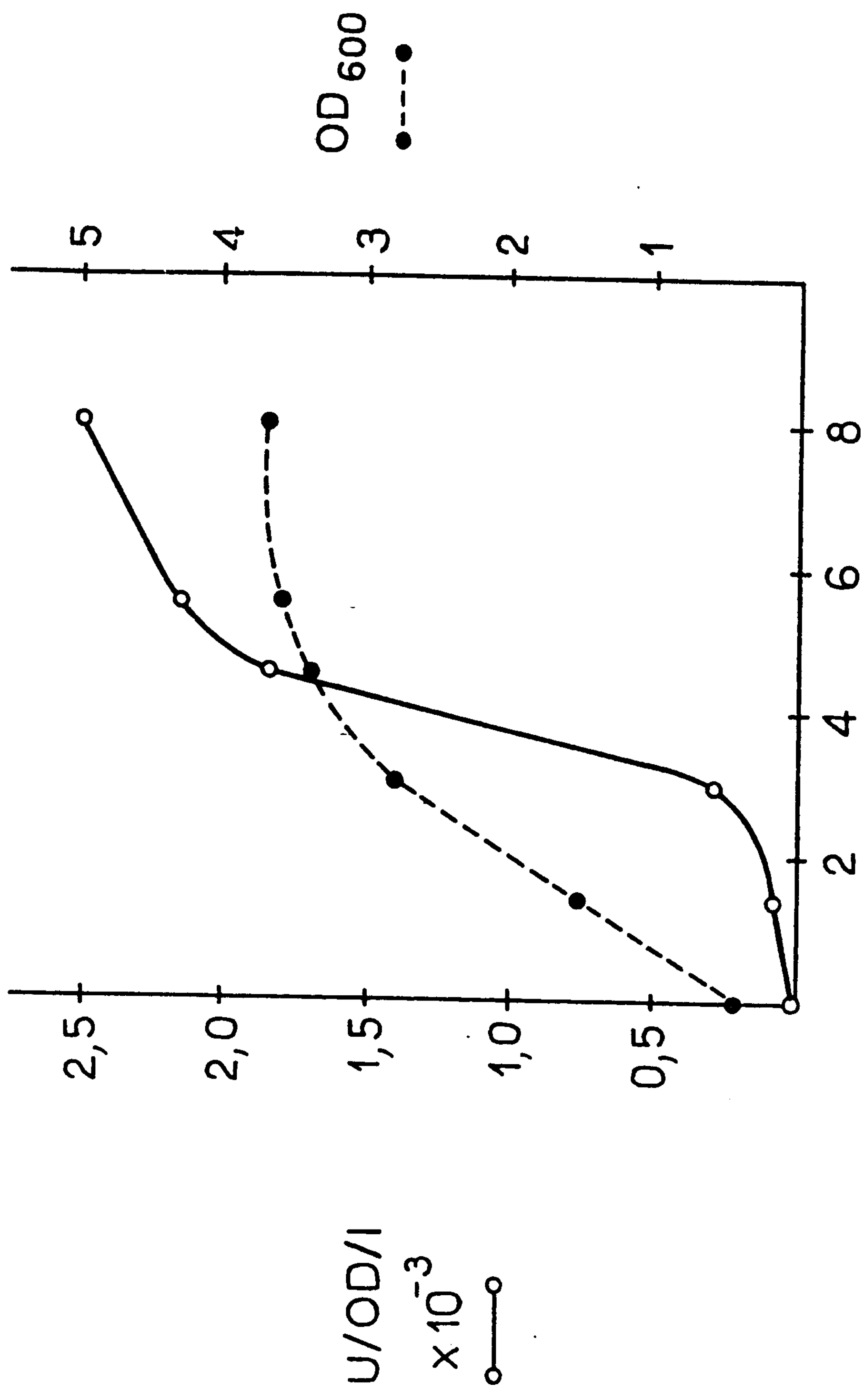


FIG_16

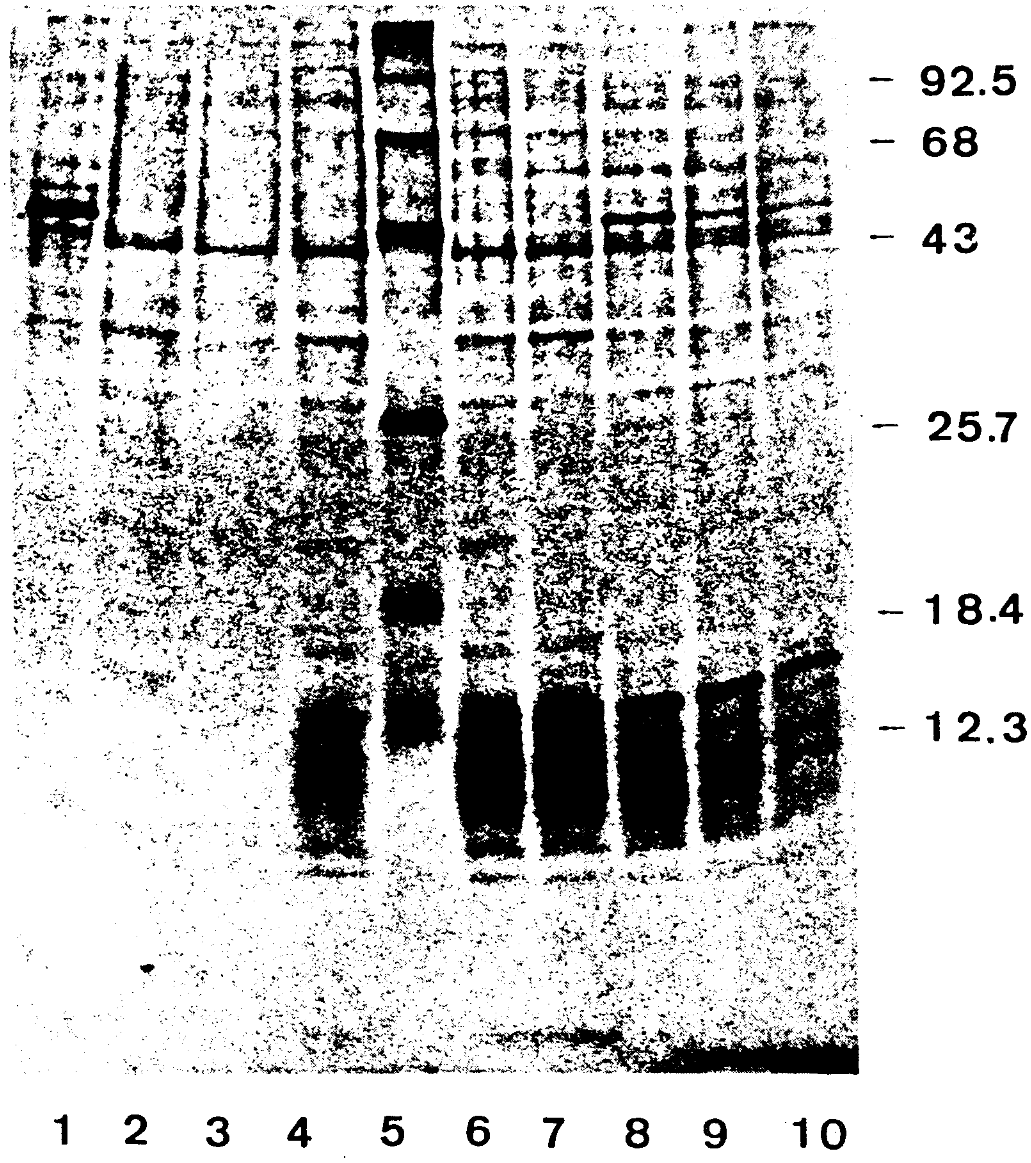


FIG_17

Pst I

a) ↓(G)ₙGCA ATC TGC GTG TCT CAA GCA (1 … 21)
b) Ala Ile Cys Val Ser Gln Ala

22 … 66
a) ATT ACT TAC ACT GAT TGT ACA GAA TCG GGT CAA AAT TTG TGC CTC
b) Ile(1) Thr Tyr Thr Asp Cys Thr Glu Ser Gly Gln Asn Leu Cys Leu(15)
c) Val(1) Val(2) ---------------------------------------------------

67 … 111
TGC GAG GGA AGC AAT GTT TGC GGT AAA GGC AAT AAG TGC ATA TTG
Cys(16) Glu Gly Ser Asn Val Cys Gly Lys Gly Asn Lys Cys Ile Leu(30)
------------------------------ Gln(24) ----------------------

112 … 156
GGT TCT AAT GGA AAG GGC AAC CAA TGT GTC ACT GGC GAA GGT ACA
Gly(31) Ser Asn Gly Lys Gly Asn Gln Cys Val Thr Gly Glu Gly Thr(45)
------- Asp(33) --- Glu(35) Lys(36) ----------------------------------

Taq I

157 … 201
CCG AAC CCT GAA AGC CAT AAT AAC GGC GAT T↓TC GAA GAA ATT CCA
Pro(46) Asn Pro Glu Ser His Asn Asn Gly Asp Phe Glu Glu Ile Pro(60)
--- Lys(47) --- Gln(49) ----------- Asp(53) ---------------------------

Aha III

202 … 256↓
GAA GAA TAT TTA CAA TGAAAAATGAAAGAATATCAATCATAGAGAATTTTGATTT
Glu(61) Glu Tyr Leu Gln(65)
------------------

257 … 316
AAAAACATTTCCATAGCTAAGCTATTTACCAATAAATAAATTAATTTTTCCATTGAATCT

317 … 376
CAATCATATTTACTCTCAATCATATTCAGCTATTTACCAATAAATAAATTAATTTTTCCA

Pst I

377
TGA(C)ₙ ↓

FIG.18

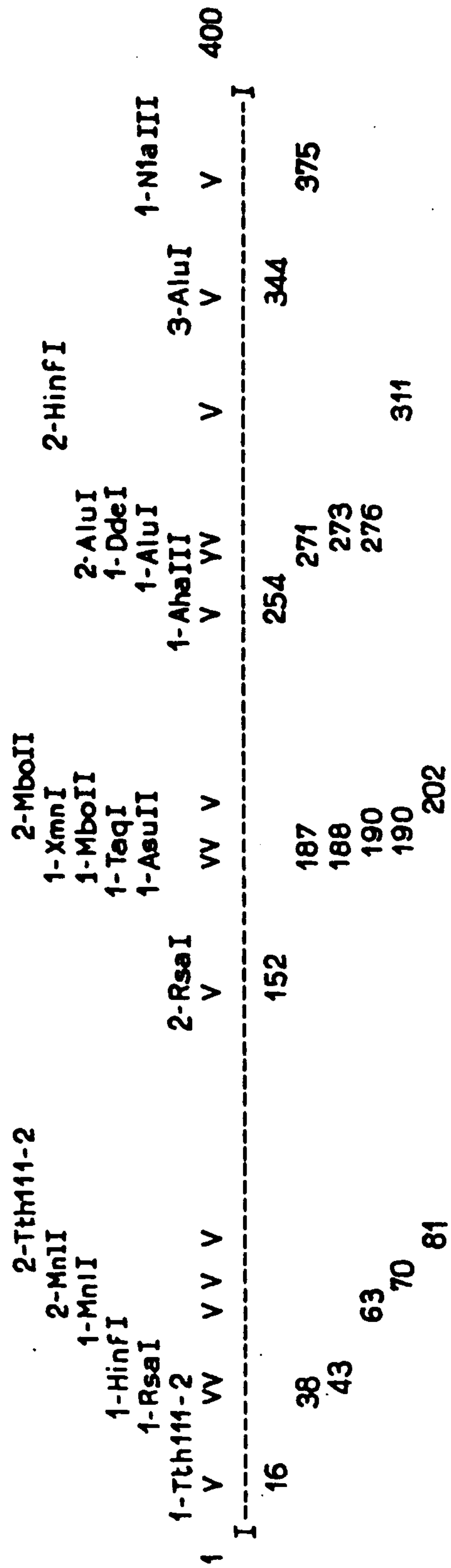
1
400
1-Tth111-2
1-RsaI
1-HinfI
1-MnlI
2-MnlI
2-Tth111-2
16
38
43
63
70
81
2-RsaI
152
1-AsuII
1-TaqI
1-MboII
1-XmnI
2-MboII
187
188
190
190
202
1-AhaIII
1-AluI
1-DdeI
2-AluI
254
271
273
276
2-HinfI
311
3-AluI
344
1-NlaIII
375

FIG.19

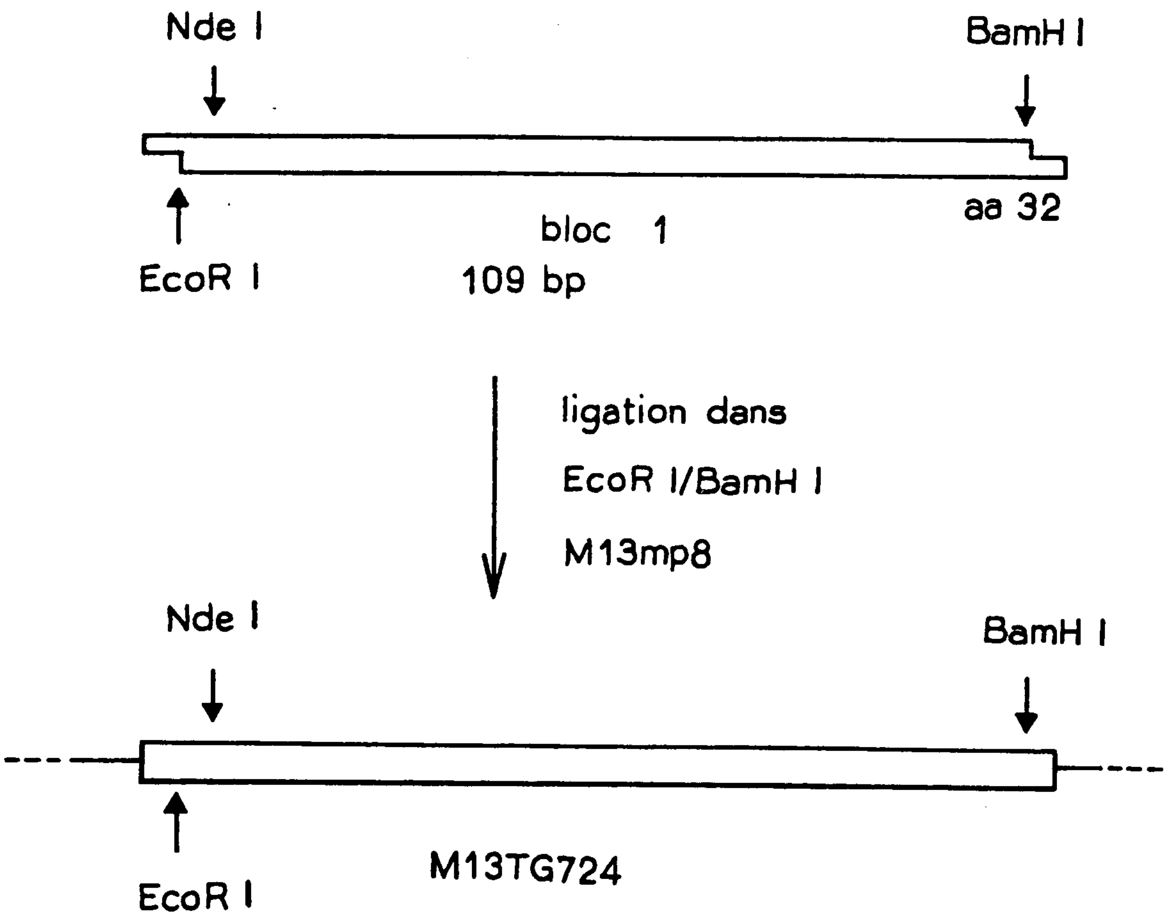
Nde I
BamH I
aa 32
bloc 1
EcoR I
109 bp
ligation dans
EcoR I/BamH I
M13mp8
Nde I
BamH I
EcoR I
M13TG724

FIG. 20

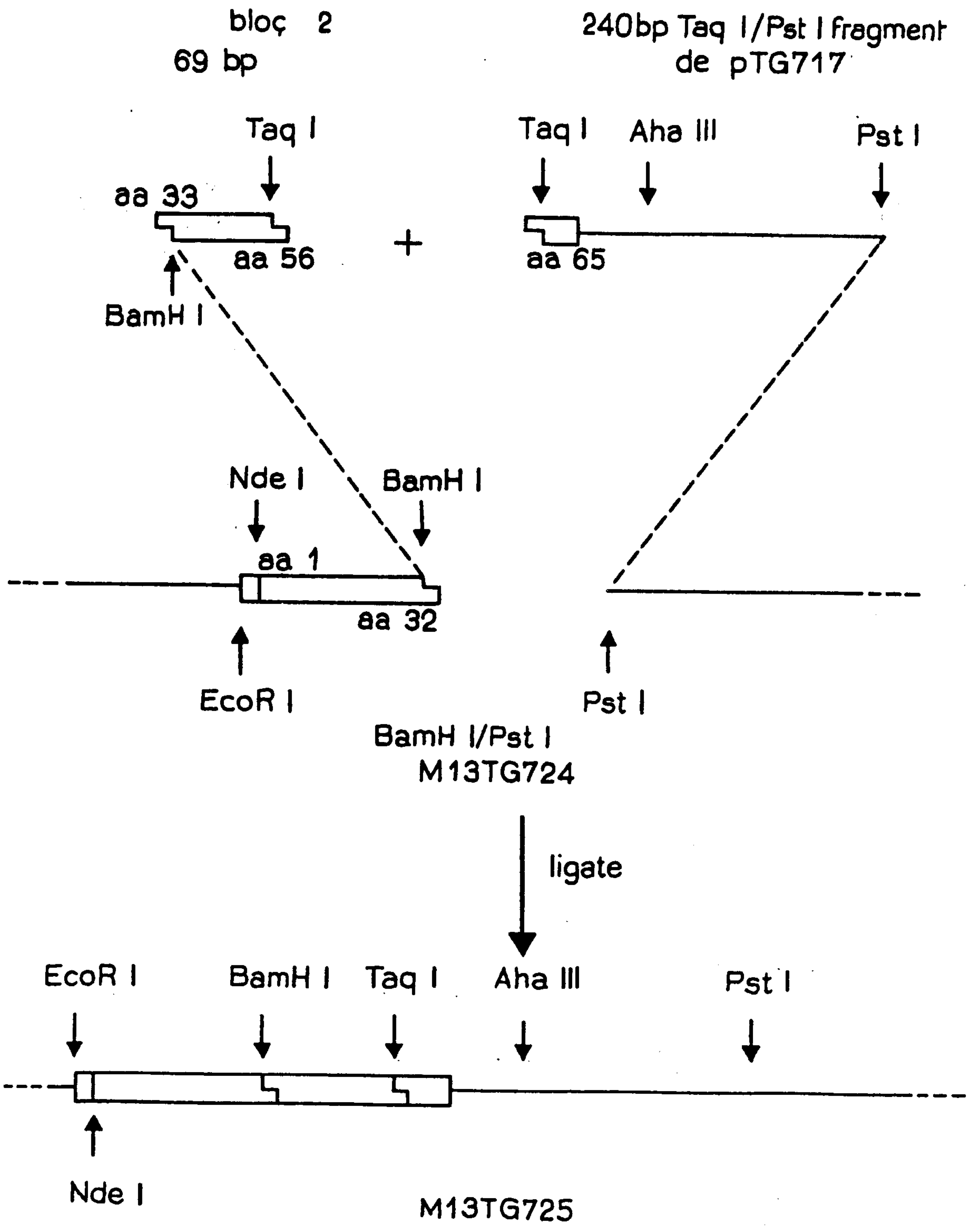
bloç 2
69 bp
240bp Taq I/Pst I fragment
de pTG717
Taq I
aa 33
aa 56
BamH I
+
Taq I
Aha III
Pst I
aa 65
Nde I
BamH I
aa 1
aa 32
EcoR I
Pst I
BamH I/Pst I
M13TG724
ligate
EcoR I
BamH I
Taq I
Aha III
Pst I
Nde I
M13TG725

FIG. 21

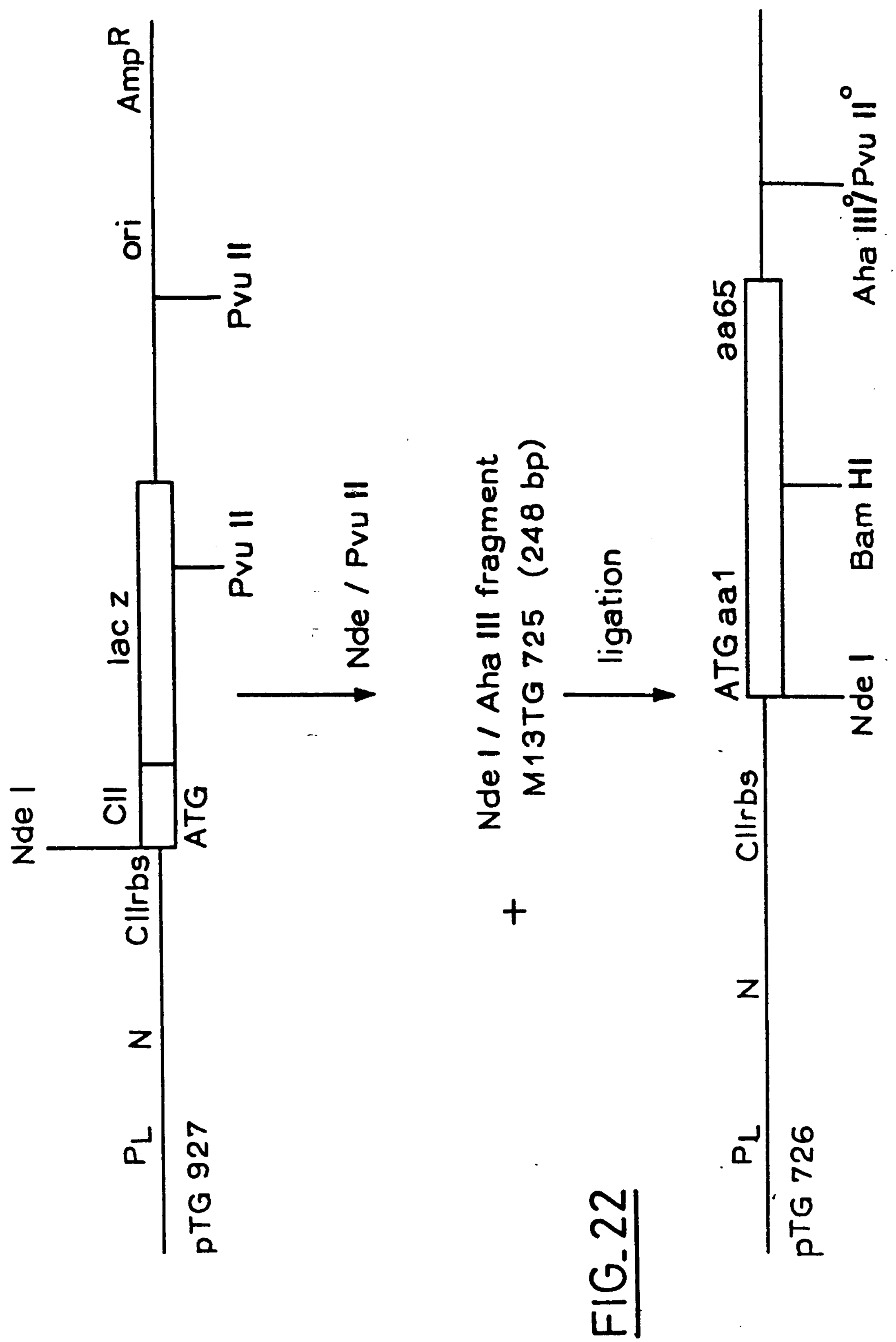

pTG 927
P_L
N
CIIrbs
Nde I
CII
ATG
lac z
Pvu II
ori
Pvu II
AmpR
Nde / Pvu II
+
Nde I / Aha III fragment
M13TG 725 (248 bp)
ligation
pTG 726
P_L
N
CIIrbs
ATG aa1
Nde I
Bam HI
aa65
Aha III°/Pvu II°


FIG. 22

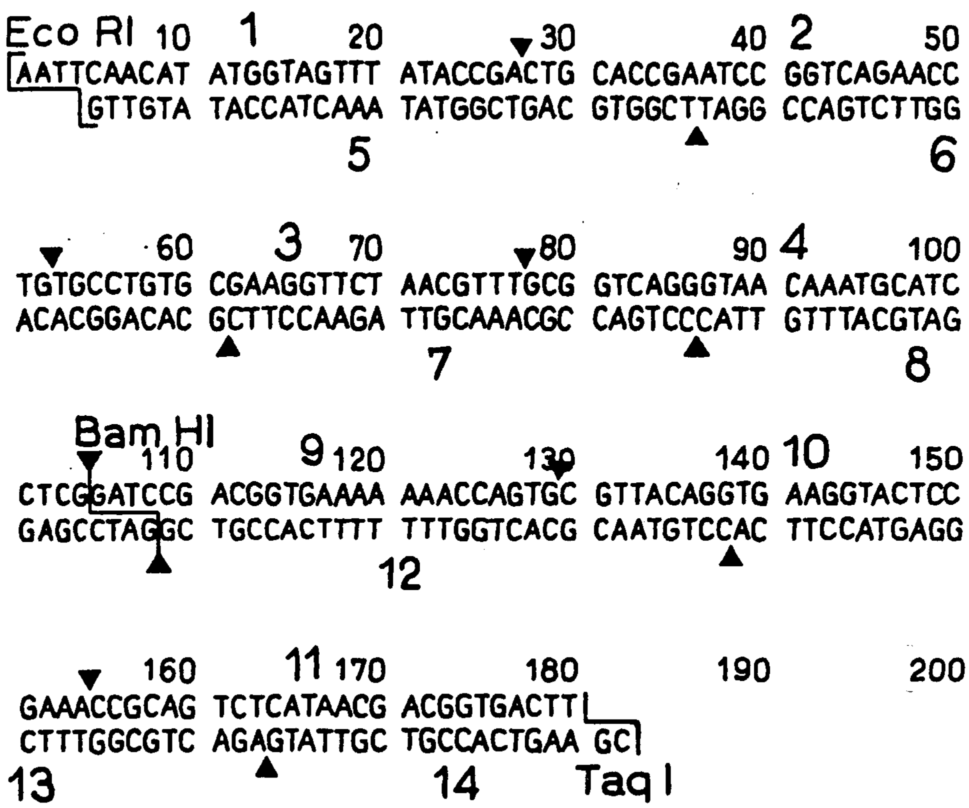


Oligonucleotides

| | | | |
|---|---|---|---|
| **Bloc 1** | | **Bloc 2** | |
| 1 | 27 bases | 9 | 25 |
| 2 | 25 | 10 | 25 |
| 3 | 25 | 11 | 26 |
| 4 | 27 | 12 | 30 |
| 5 | 32 | 13 | 25 |
| 6 | 25 | 14 | 19 |
| 7 | 25 | | |
| 8 | 22 | | |

FIG_23

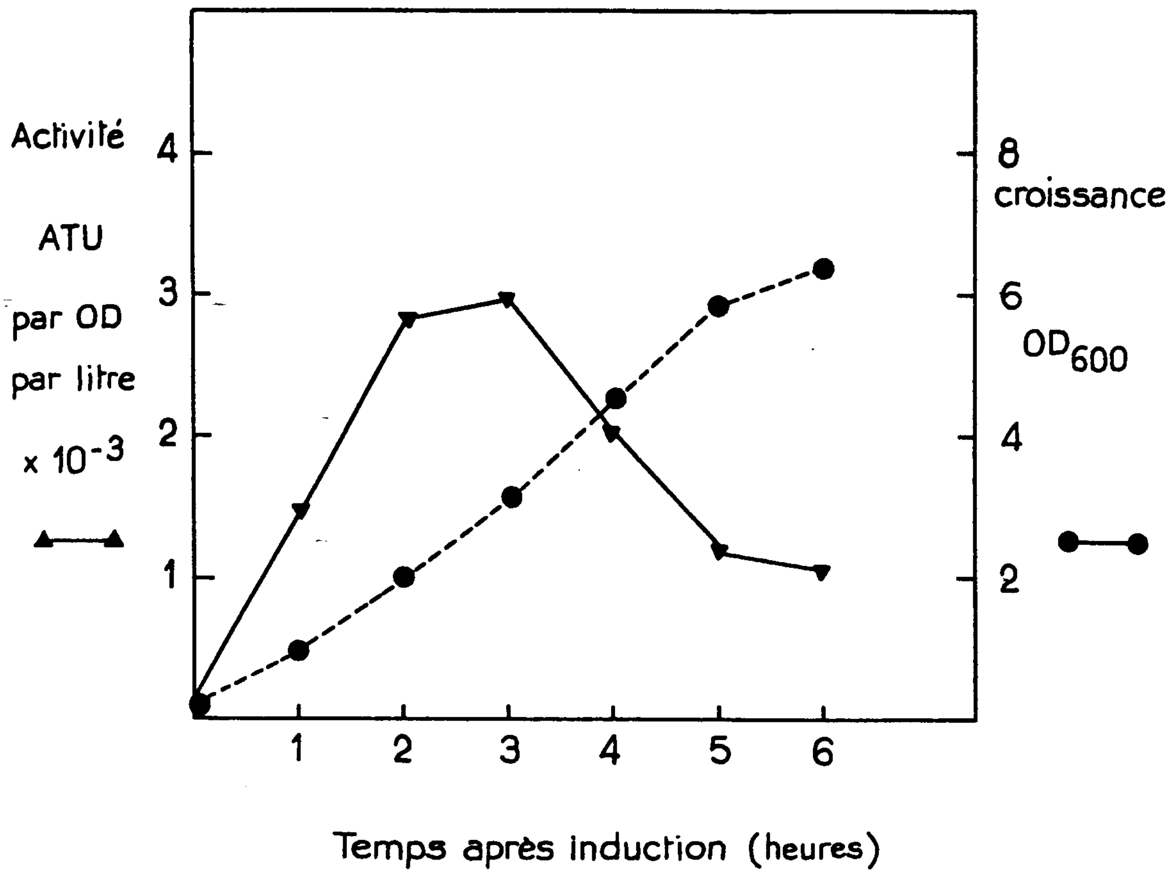
Activité
ATU
par OD
par litre
x $10^{-3}$
4
3
2
1
8
croissance
6
$OD_{600}$
4
2
1
2
3
4
5
6
Temps après induction (heures)

FIG_24

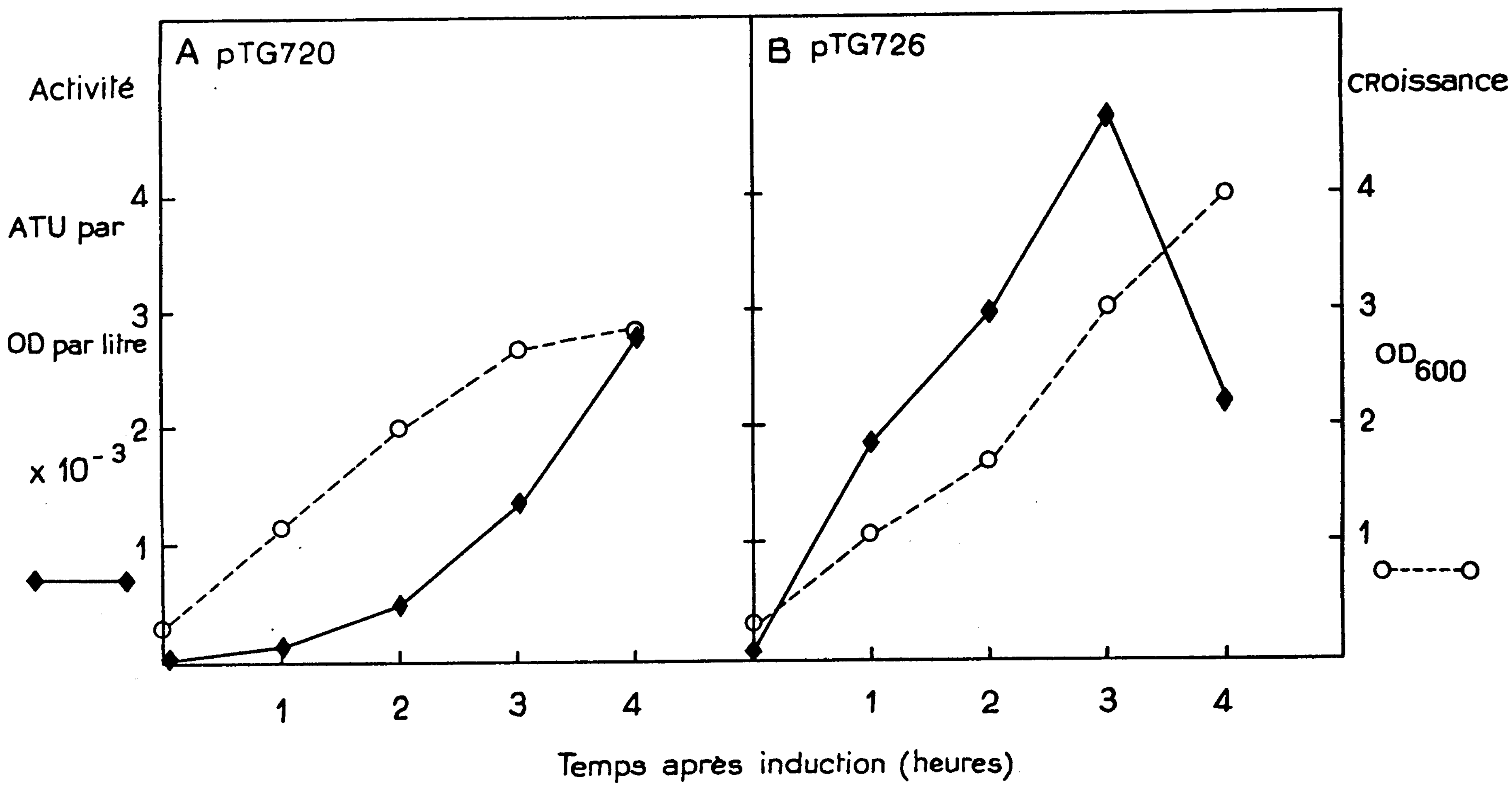
A pTG720
B pTG726
Activité
ATU par
OD par litre
x 10⁻³
croissance
OD600
1
2
3
4
Temps après induction (heures)

FIG. 25